(19) Europäisches Patentamt
European Patent Office
Office européen des brevets

(11) **EP 1 877 576 B1**

(12) **EUROPEAN PATENT SPECIFICATION**

(45) Date of publication and mention of the grant of the patent:
**23.01.2013 Bulletin 2013/04**

(21) Application number: **06749954.1**

(22) Date of filing: **12.04.2006**

(51) Int Cl.:
***C12Q 1/68*** (2006.01)

(86) International application number:
**PCT/US2006/013753**

(87) International publication number:
**WO 2006/110855 (19.10.2006 Gazette 2006/42)**

(54) **METHODS FOR DETERMINING SEQUENCE VARIANTS USING ULTRA-DEEP SEQUENCING**

VERFAHREN ZUR BESTIMMUNG VON SEQUENZVARIANTEN MITTELS ULTRA-DEEP-SEQUENZIERUNG

PROCEDES DE DETERMINATION DE VARIANTES DE SEQUENCE UTILISANT UN SEQUENCAGE DES AMPLICONS

(84) Designated Contracting States:
**AT BE BG CH CY CZ DE DK EE ES FI FR GB GR HU IE IS IT LI LT LU LV MC NL PL PT RO SE SI SK TR**

(30) Priority: **12.04.2005 US 104781**
**06.06.2005 US 688042 P**

(43) Date of publication of application:
**16.01.2008 Bulletin 2008/03**

(60) Divisional application:
**10179666.2 / 2 341 151**

(73) Proprietor: **454 Life Sciences Corporation**
**Branford CT 06405 (US)**

(72) Inventors:
• **LEAMON, John, Harris**
**Guilford, Connecticut 06437 (US)**
• **LEE, William, Lun**
**Madison, Connecticut 06443 (US)**
• **SIMONS, Jan, Fredrick**
**California 94010 (US)**
• **DESANY, Brian**
**Brandford, Connecticut 06405 (US)**
• **RONAN, Mike, Todd**
**New Haven, Connecticut 06513 (US)**
• **DRAKE, James**
**Branford, Connecticut 06405 (US)**
• **LOHMAN, Kenton**
**Guilford, Connecticut 06437 (US)**
• **EGHOLM, Michael**
**Madison, Connecticut 06443 (US)**
• **ROTHBERG, Jonathan**
**Guilford, Connecticut 06437 (US)**

(74) Representative: **Ireland, Jacqueline Frances et al**
**Mintz Levin Cohn Ferris Glovsky and**
**Popeo Intellectual Property LLP**
**Alder Castle**
**10 Noble Street**
**London EC2V 7JX (GB)**

(56) References cited:
**WO-A-2005/003375     WO-A2-01/68897**
**WO-A2-2004/069849**

• **FAKHRAI-RAD HOSSEIN ET AL:** "Pyrosequencing: an accurate detection platform for single nucleotide polymorphisms." HUMAN MUTATION. MAY 2002, vol. 19, no. 5, May 2002 (2002-05), pages 479-485, XP002408034 ISSN: 1098-1004
• **SHIFMAN SAGIV ET AL:** "Quantitative technologies for allele frequency estimation of SNPs in DNA pools." MOLECULAR AND CELLULAR PROBES, vol. 16, no. 6, December 2002 (2002-12), pages 429-434, XP002408036 ISSN: 0890-8508
• **LI ZENGMIN ET AL:** "A photocleavable fluorescent nucleotide for DNA sequencing and analysis." PROCEEDINGS OF THE NATIONAL ACADEMY OF SCIENCES OF THE UNITED STATES OF AMERICA. 21 JAN 2003, vol. 100, no. 2, 21 January 2003 (2003-01-21), pages 414-419, XP002408035 ISSN: 0027-8424

- **DA MOTA A F ET AL: "Distribution of bovine lymphocyte antigen (BoLA-DRB3) alleles in Brazilian dairy Gir cattle (Bos indicus).", EUROPEAN JOURNAL OF IMMUNOGENETICS : OFFICIAL JOURNAL OF THE BRITISH SOCIETY FOR HISTOCOMPATIBILITY AND IMMUNOGENETICS JUN 2002 LNKD- PUBMED: 12047358, vol. 29, no. 3, June 2002 (2002-06), pages 223-227, ISSN: 0960-7420**

SEQUENCE LISTING

<110> Leamon, John H
Lee, William L
Simons, Jan F
Desany, Brian
Ronan, Mike T
Drake, James
Lohman, Kenton
Egholm, Michael
Rothberg, Jonathan

<120> Methods For Determining Sequence variants using ultra-Deep sequencing

<130> 21465-515 UTIL

<140> 11/104,781
<141> 2005-04-12

<160> 34

<170> PatentIn version 3.3

<210> 1
<211> 41
<212> DNA
<213> Artificial

<220>
<223> synthesized oligonucleotides

<400> 1
gcctccctcg cgccatcaga cctccctctg tgtccttaca a          41

<210> 2
<211> 41
<212> DNA
<213> Artificial

<220>
<223> synthesized oligonucleotides

<400> 2
gccttgccag cccgctcagg gagggaatca tactagcacc a          41

<210> 3
<211> 43
<212> DNA
<213> Artificial

<220>
<223> synthesized oligonucleotides

<400> 3
gcctccctcg cgccatcagt ctgacgatct ctgtcttcta acc          43

<210> 4
<211> 39
<212> DNA
<213> Artificial

<220>
<223> Synthesized oligonucleotides

<400> 4
gccttgccag cccgctcagg ccttgaacta cacgtggct        39

<210> 5
<211> 39
<212> DNA
<213> Artificial

<220>
<223> Synthesized oligonucleotides

<400> 5
gcctccctcg cgccatcaga tttctctacc acccctggc        39

<210> 6
<211> 39
<212> DNA
<213> Artificial

<220>
<223> synthesized oligonucleotides

<400> 6
gccttgccag cccgctcaga gctcatgtct cccgaagaa        39

<210> 7
<211> 39
<212> DNA
<213> Artificial

<220>
<223> Synthesized oligonucleotides

<400> 7
gcctccctcg cgccatcaga aagccagaag aggaaaggc        39

<210> 8
<211> 39
<212> DNA
<213> Artificial

<220>
<223> Synthesized oligonucleotides

<400> 8
gccttgccag cccgctcagc ttgcagattg gtcataagg        39

<210> 9
<211> 39
<212> DNA
<213> Artificial

<220>
<223> Synthesized oligonucleotides

<400> 9
gcctccctcg cgccatcaga cagtgcaaac accaccaaa          39


<210> 10
<211> 39
<212> DNA
<213> Artificial


<220>
<223> Synthesized oligonucleotides


<400> 10
gccttgccag cccgctcagc cagtattcat ggcagggtt          39


<210> 11
<211> 15
<212> DNA
<213> Artificial


<220>
<223> Synthesized oligonucleotides


<400> 11
gcctccctcg cgcca          15


<210> 12
<211> 15
<212> DNA
<213> Artificial


<220>
<223> Synthesized oligonucleotides


<400> 12
gccttgccag cccgc          15


<210> 13
<211> 41
<212> DNA
<213> Artificial


<220>
<223> Synthesized oligonucleotides


<400> 13
gcctccctcg cgccatcagg aagagtttga tcatggctca g          41


<210> 14
<211> 39
<212> DNA
<213> Artificial


<220>
<223> Synthesized oligonucleotides


<400> 14
gccttgccag cccgctcagt tactcacccg tccgccact          39

<210> 15
<211> 39
<212> DNA
<213> Artificial

<220>
<223> Synthesized Oligonucleotides

<400> 15
gcctccctcg cgccatcagg caacgcgaag aaccttacc          39

<210> 16
<211> 39
<212> DNA
<213> Artificial

<220>
<223> synthesized oligonucleotides

<400> 16
gccttgccag cccgctcaga cgacagccat gcagcacct          39

<210> 17
<211> 72
<212> DNA
<213> Artificial

<220>
<223> Synthesized oligonucleotides

<400> 17

```
aagagttttg atcatggctc agattgaacg ctggcggcag gcctaacaca tgcaagtcga          60
acggtaacag ga          72
```

<210> 18
<211> 71
<212> DNA
<213> Escherichia coli

<400> 18

```
tttgatcatg gctcagattg aacgctggcg gcaggcctaa cacatgcaag tcgaacggta          60
acgaggaacg a          71
```

<210> 19
<211> 70
<212> DNA
<213> Escherichia coli

<400> 19

```
tttgatcatg gctcagattg aacgctggcg gcaggcctaa cacatgcaag tcgaacggta          60
acaggaacga          70
```

<210> 20
<211> 72
<212> DNA
<213> Artificial

<220>
<223> Synthesized Oligonucleotides

<400> 20

```
aagagttttg atcatggctc agattgaacg ctggcggcag gcctaacaca tgcaagtcga    60

acggtaacag ga    72
```

<210> 21
<211> 71
<212> DNA
<213> Escherichia coli

<400> 21

```
aagagtttga tcatggctca gattgaacgc tggcggcagg cctaacacat gcaagtcgaa    60

cggtaacagg a    71
```

<210> 22
<211> 71
<212> DNA
<213> Escherichia coli

<400> 22

```
aagagtttga tcatggctca gattgaacgc tggcggcagg cctaacacat gcaagtcgaa    60

cggtaacagg a    71
```

<210> 23
<211> 104
<212> DNA
<213> Artificial

<220>
<223> Synthesized oligonucleotides

<400> 23

```
caacgcgaag aaccttacct ggtcttgaca tccacgaagt ttactagaga tgagaatgtg    60

ccgttcggga accggtgaga caggtgctgc atggctgtcg tctg    104
```

<210> 24
<211> 102
<212> DNA
<213> Escherichia coli

<400> 24

```
caacgcgaag aaccttacct ggtcttgaca tccacgaagt ttactagaga tgagaatgtg     60

ccgttcggga accggtgaga caggtgctgc atggctgtcg tc                         102
```

<210> 25
<211> 99
<212> DNA
<213> Escherichia coli

<400> 25

```
caacgcgaag aaccttacct ggtcttgaca tccacgaagt tttcagagat gagaatgtgc     60

cttcgggaac cgtgagacag gtgctgcatg gctgtcgtc                             99
```

<210> 26
<211> 102
<212> DNA
<213> Artificial

<220>
<223> Synthesized oligonucleotides

<400> 26

```
caacgcgaag aaccttacct ggtcttgaca tccacgaagt ttacagagat gagaatgtgc     60

cgttcgggaa ccgtgagaca ggtgctgcat ggctgtcgtc tg                         102
```

<210> 27
<211> 100
<212> DNA
<213> Escherichia coli

<400> 27

```
caacgcgaag aaccttacct ggtcttgaca tccacgaagt ttacagagat gagaatgtgc     60

cgttcgggaa ccgtgagaca ggtgctgcat ggctgtcgtc                            100
```

<210> 28
<211> 99
<212> DNA
<213> Escherichia coli

<400> 28

```
caacgcgaag aaccttacct ggtcttgaca tccacgaagt tttcagagat gagaatgtgc     60

cttcgggaac cgtgagacag gtgctgcatg gctgtcgtc                             99
```

<210> 29
<211> 5
<212> DNA
<213> Artificial

<220>
<223> Synthesized oligonucleotides

<400> 29
aaaaa            5


<210> 30
<211> 20
<212> DNA
<213> Artificial

<220>
<223> Synthesized oligonucleotides

<400> 30
ccatctgttg cgtgcgtgtc         20


<210> 31
<211> 20
<212> DNA
<213> Artificial

<220>
<223> synthesized oligonucleotides

<400> 31
cgtttcccct gtgtgccttg         20


<210> 32
<211> 20
<212> DNA
<213> Artificial

<220>
<223> synthesized Oligonucleotides

<400> 32
ccatctgttg cgtgcgtgtc         20


<210> 33
<211> 40
<212> DNA
<213> Artificial

<220>
<223> Synthesized Oligonucleotides

<400> 33
cgtttcccct gtgtgccttg ccatctgttc cctccctgtc          40


<210> 34
<211> 15
<212> DNA
<213> Artificial

<220>
<223> Synthesized oligonucleotide

<400> 34
gcctccctcg cgcca            15

## Description

## FIELD OF THE INVENTION

The invention provides a method for detecting and analyzing low frequency sequence variants including single nucleotide polymorphisms (SNPs), insertion/deletion variant (referred to as "indels") and allelic frequencies, in a population of target polynucleotides in parallel.

## BACKGROUND OF THE INVENTION

Genomic DNA varies significantly from individual to individual, except in identical siblings. Many human diseases arise from genomic variations. The genetic diversity amongst humans and other life forms explains the heritable variations observed in disease susceptibility. Diseases arising from such genetic variations include Huntington's disease, cystic fibrosis, Duchenne muscular dystrophy, and certain forms of breast cancer. Each of these diseases is associated with a single gene mutation. Diseases such as multiple sclerosis, diabetes, Parkinson's, Alzheimer's disease, and hypertension are much more complex. These diseases may be due to polygenic (multiple gene influences) or multifactorial (multiple gene and environmental influences) causes. Many of the variations in the genome do not result in a disease trait. However, as described above, a single mutation can result in a disease trait. The ability to scan the human genome to identify the location of genes which underlie or are associated with the pathology of such diseases is an enormously powerful tool in medicine and human biology.

Several types of sequence variations, including insertions and deletions (indels), differences in the number of repeated sequences, and single base pair differences (SNPs) result in genomic diversity. Single base pair differences, referred to as single nucleotide polymorphisms (SNPs) are the most frequent type of variation in the human genome (occurring at approximately 1 in $10^3$ bases). As used herein, a SNP can be any genomic position at which at least two or more alternative nucleotide alleles occur. As used herein, a SNP may also refer to any single base insertion/deletion variant (referred to as "indel"), or an indel involving the insertion and/or deletion of between 2 and 100 or more bases. SNPs are well-suited for studying sequence variation because they are relatively stable (i.e., exhibit low mutation rates) and because they can be responsible for inherited traits. It is understood that in the discussion above, the term SNP is also meant to be applicable to "indel" (defined below).

Polymorphisms identified using microsatellite-based analysis, for example, have been used for a variety of purposes. Use of genetic linkage strategies to identify the locations of single Mendelian factors has been successful in many cases (Benomar et al. (1995), Nat. Genet., 10:84-8; Blanton et al. (1991), Genomics, 11:857-69). Identification of chromosomal locations of tumor suppressor genes has generally been accomplished by studying loss of heterozygosity in human tumors (Cavenee et al. (1983), Nature, 305:779-784; Collins et al. (1996), Proc. Natl. Acad Sci. USA, 93: 14771-14775; Koufos et al. (1984), Nature, 309:170-172; and Legius et al. (1993), Nat. Genet., 3:122-126). Additionally, use of genetic markers to infer the chromosomal locations of genes contributing to complex traits, such as type I diabetes (Davis et al. (1994), Nature, 371:130-136; Todd et al. (1995), Proc. Natl. Acad. Sci. USA, 92:8560-8565), has become a focus of research in human genetics.

Although substantial progress has been made in identifying the genetic basis of many human diseases, current methodologies used to develop this information are limited by prohibitive costs and the extensive amount of work required to obtain genotype information from large sample populations. These limitations make identification of complex gene mutations contributing to disorders such as diabetes extremely difficult. Techniques for scanning the human genome to identify the locations of genes involved in disease processes began in the early 1980s with the use of restriction fragment length polymorphism (RFLP) analysis, (Botstein et al. (1980), Am. J. Hum. Genet., 32:314-31; Nakamura et al. (1987), Science, 235:1616-22). RFLP analysis involves southern blotting and other techniques. Southern blotting is both expensive and time-consuming when performed on large numbers of samples, such as those required to identify a complex genotype associated with a particular phenotype. Some of these problems were avoided with the development of polymerase chain reaction (PCR) based microsatellite marker analysis. Microsatellite markers are simple sequence length polymorphisms (SSLPs) consisting of di-, tri-, and tetra-nucleotide repeats.

Other types of genomic analysis are based on use of markers which hybridize with hypervariable regions of DNA having multiallelic variation and high heterozygosity. The variable regions which are useful for fingerprinting genomic DNA are tandem repeats of a short sequence referred to as a mini satellite. Polymorphism is due to allelic differences in the number of repeats, which can arise as a result of mitotic or meiotic unequal exchanges or by DNA slippage during replication.

Currently, the identification of variations by DNA sequencing is hampered by a number of shortcomings. In current methods, the amplification of a region of interest is followed by direct sequencing of the amplification product (i.e. a mix of variant sequences). Alternatively, the sequencing step is preceded by a microbial subcloning step, i.e. by recombinant insertion of amplification products into a vector suitable for propagation in the intended host organism.

The disadvantage of direct sequencing of the amplification product lies in a mixed signal occurring at variable sites in the sequence. The relative contributions of the different nucleotides in such mixed signals are difficult or impossible to quantitate, even when the frequency of the lower abundance allele approaches 50%. Furthermore, if the variation is an insertion or deletion (rather than a base substitution), the resulting phase shift between the different molecules will lead to a scrambled, unreadable signal.

The addition of a microbial cloning step overcomes the problems associated with direct sequencing, in that mixed signals are not encountered. However, this strategy requires a larger number of sequencing reactions. Furthermore, the microbial cloning step is expensive and time consuming, and may also select against certain variants, and thus skewing the relative frequency of the variants. If sequencing of a large number (i.e. hundreds, thousands, tens of thousands) of clones is desired, the cost becomes extremely high.

Each of these current methods have significant drawbacks because they are time consuming and limited in resolution. While DNA sequencing provides the highest resolution, it is also the most expensive method for determining SNPs. At this time, the determination of SNP frequency among a population of 1000 different samples is very expensive and the determination of SNP frequency among a population of 100,000 samples is prohibitive. Thus, a continuing need exists in the art for economical methods of identification and resequencing of sequence variants present in polynucleotide populations, especially variants present at low frequencies.

Da Mota et al. (2002), Eur. J. Immunogen, 29:223-227 describes two experimental genotyping approaches to identify alleles of the BoLA-DRB3 gene from tropical zebu dairy breed cattle: (i) direct sequencing of PCR gene products; and (ii) sequencing of cloned PCR fragments.

## BRIEF SUMMARY OF THE INVENTION

In accordance with the present invention there is provided a method for detecting sequence variants having a frequency of less than 5% in a nucleic acid population, the method comprising the steps of:

(a) amplifying a polynucleotide segment common to said nucleic acid population with a pair of PCR nucleic acid primers that define a locus to produce a first population of amplicons each comprising said polynucleotide segment;
(b) delivering the first population of amplicons into aqueous microreactors in a water-in-oil emulsion such that a plurality of the aqueous microreactors comprise (1) a single amplicon of the first population of amplicons (2) a single bead, and (3) amplification reaction solution containing reagents necessary to perform nucleic acid amplification;
(c) clonally amplifying each member of said first population of amplicons by polymerase chain reaction to produce a plurality of populations of second amplicons wherein each population of second amplicons derives from one member of said first population of amplicons;
(d) immobilizing said second amplicons to a plurality of the beads in the microreactors such that each bead comprises one population of said second amplicons;
(e) breaking the emulsion to recover the beads from the microreactors;
(f) determining in parallel a nucleic acid sequence for the second amplicons on each bead at a depth (i.e. number of individual sequence reads) of greater than 100 to produce a population of nucleic acid sequences; and
(g) determining an incidence of each type of nucleotide at each position of said polynucleotide segment to detect said sequence variants in said nucleic acid population;

wherein the method does not require prior knowledge of the nucleic acid sequence composition of the sequence variants, and wherein said nucleic acid primers are bipartite primers comprising a 5' region and a 3' region, wherein said 3' region is complementary to a region on said polynucleotide segment and wherein said 5' region is homologous to a sequencing primer or complement thereof.

In accordance with the invention, there is also provided a method of identifying a distribution of organisms in a population comprising a plurality of different individual organisms, the method comprising using a nucleic acid sample from said population and comprising the steps of:

(a) determining sequence variants of a nucleic acid segment comprising a locus common to all organisms in said population using the above-mentioned method of the invention, wherein each organism comprises a different nucleic acid sequence at said locus; and
(b) identifying the distribution of organisms in said population based on said population of nucleic acid sequences.

Also in accordance with the invention, there is provided a method for determining a composition of a tissue sample, the method comprising using a nucleic acid sample from said tissue sample and comprising the steps of:

(a) detecting sequence variants of a nucleic acid segment using the above-mentioned method of the invention, wherein said segment comprises a locus common to all cells in said tissue sample and wherein each cell type comprises a different sequence variant at said locus; and

(b) determining the composition of said tissue sample from said nucleotide frequency. The terms "polynucleotide region" and "locus" are used interchangeably herein. The amplification and sequencing methods of the present invention facilitate single molecule sequencing. This single molecule resolution allows highly accurate detection and/or frequency measurement of variations that are present at very low frequencies in a template polynucleotide mixture.

The present invention enables the exact measurement of sequence variations among nucleic acid mixtures, especially variations occurring at low or very low frequency. The inclusion of an amplification step targeted at a specific region of interest in a nucleic acid sample, coupled with so-called single molecule sequencing technologies, allows for accurate, fast and low-cost discovery of sequence variants, and measurement of allele frequencies. This improvement over previously known methods is achieved, in part, by the use of a sequence specific *in vitro* amplification step preceding single-molecule sequencing.

A salient feature of the present invention is the capability to determine the nucleotide sequence of a polynucleotide region of interest at great depth. By depth is meant the number of individual sequence reads spanning a given region of interest. For example, if 1000 molecules are sequenced separately, the depth equals 1000, and may also be referred to as "1000-fold" or "1000 X". According to the invention, the depth may range from about 100 to about several billion, for example from about 100 to about 1 million, from about 100 to about 10 million, from about 100 to about 100,000, or from about 1000 to about 1 million. The depth may be greater than about 100, greater than about 1000, greater than about 10,000, greater than about 100,000, greater than about 1 million, greater than about 10 million, greater than about 100 million, greater than about 1 billion. The sequence depth achieved by the methods of the present invention is much greater than the depths achievable, practical or affordable by current methods. Specifically, the methods of the current invention do not require microbial cloning. By microbial cloning is meant the amplification of polynucleotides in microbial host organisms, for example E. coli. It will be apparent to the skilled artisan that the depths made possible by the present invention facilitate the detection of rare sequence variants with relative ease, speed and at low cost.

The invention relates to the detection of a number of sequence variants (e.g., allelic variants, single nucleotide polymorphism variants, indel variants) by the identification of specific polynucleotide sequences. Current technology allows detection of SNPs, for example, by polymerase chain reaction (PCR). However, SNP detection by PCR requires the design of special PCR primers which hybridize to one type of SNP and not another type of SNP. Furthermore, although PCR is a powerful technique, the specific PCR of alleles require prior knowledge of the nature (sequence) of the SNP, as well as multiple PCR runs and analysis on gel electrophoresis to determine an allelic frequency. For example, an allelic frequency of 5% (i.e., 1 in 20) would require at a minimum 20 PCR reactions for its detection. The amount of PCR and gel electrophoresis needed to detect an allelic frequency goes up dramatically as the allelic frequency is reduced, for example to 4%, 3%, 2%, 1%, 0.5 %, 0.2%, or less.

None of the current methods has provided a simple and rapid method of detecting SNP, including SNP of low abundance, by identification of specific DNA sequence.

A two stage PCR technique coupled with a novel pyrophosphate sequencing technique allows the detection of sequence variants (SNP, indels and other DNA polymorphisms) in a rapid, reliable, and cost effective manner. Furthermore, the method of the invention can detect sequence variants which are present in a DNA sample in nonstoichiometric allele amounts, such as, for example, DNA variants present in less than about 5% or less than about 1%. The techniques may conveniently be termed "ultra deep sequencing."

The term allele, as used herein, includes a sequence variation at a variable site, wherein the variation may occur within a single organism, between individual organisms of the same species, or between individuals of different species, between normal and diseased tissues derived from one or more individual, and between viral genomes.

## BRIEF DESCRIPTION OF THE FIGURES

Figure 1     depicts a schematic of one embodiment of a bead emulsion amplification process.

Figure 2     depicts a schematic of one embodiment of the ultradeep sequencing method.

Figure 3     depicts quality assessment of amplicons produced with primer pairs SAD1F/R-DD14 (panel A), SAD1F/R-DE15 (panel B) and SAD1F/R-F5 (panel C). Analysis was performed on a BioAnalyzer DNA 1000 BioChip with the center peaks representing the PCR products and the flanking peaks reference size markers. Each peak was measured to be within 5 bp of the theoretical size which ranged from 156-181 base pairs.

Figure 4     depicts nucleotide frequencies (frequency of non-matches) in amplicons representing two distinct alleles in the MHC II locus were mixed in approximate ratios (C allele to T allele) of 1:500 (A) and 1:1000 (B), or T allele only (A), clonally amplified and sequenced on 454 Life Sciences' sequencing platform. Each bar represents the frequency of deviation from the consensus sequence and are color-coded according to the resulting base substitution (red=A; green=C; blue=G; yellow=T).

Figure 5    depicts the same data as presented in Figure 4B and 4C, however after background subtraction using the T allele only sample presented in Figure 4A.

Figure 6    depicts various ratios of C to T alleles from the DD14 HLA locus were mixed and sequenced on the 454 platform to determine dynamic range. The experimentally observed ratios are plotted against the intended ratios (abscissa). The actual number of sequencing reads for each data point is summarized in Table 1

Figure 7    A: A graphical display showing the location of the reads mapping to the 1.6 Kb 16S gene fragment indicating roughly 12,000 reads mapping to the first 100 bases of the 16S gene. B: shows similar results as 7A except with the V3 primers which maps to a region around base 1000. C: shows locations of the reads where both V1 and V3 primers are used.

Figure 8    depicts a phylogenetic tree which clearly discriminates between the V1 (shorter length on left half of figure) and the V3 (longer length on right half of figure) sequences in all but 1 of the 200 sequences.

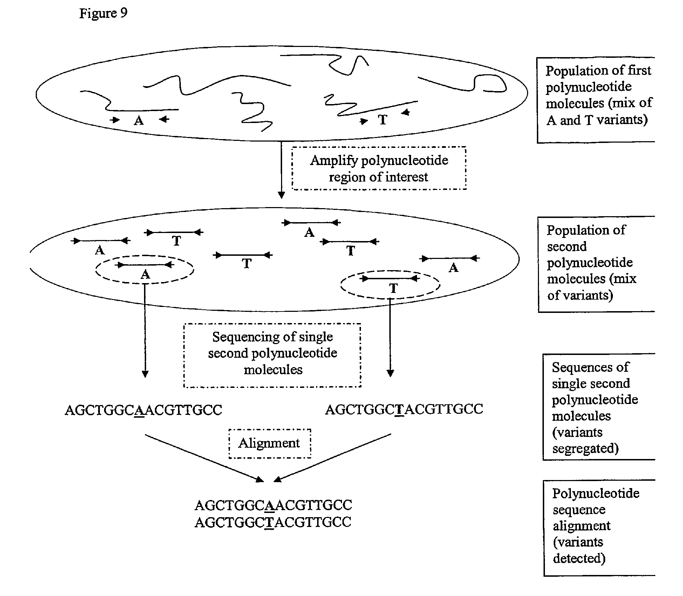

Figure 9    depicts a schematic of one embodiment of the ultra deep sequencing method. Horizontal arrows depict primers flanking the region of interest.

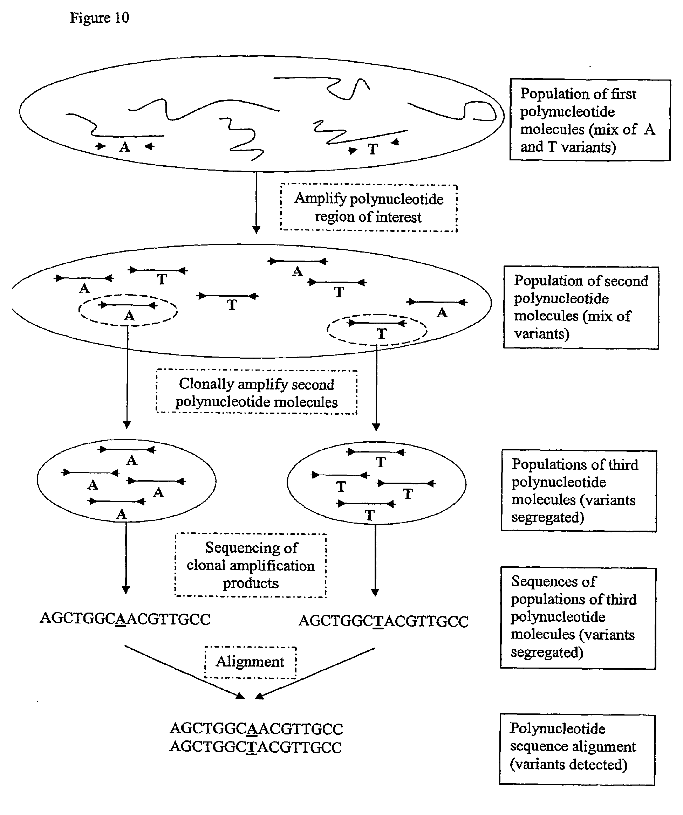

Figure 10   depicts a schematic of another embodiment of the ultra deep sequencing method. Horizontal arrows depict primers flanking the region of interest.

## DETAILED DESCRIPTION OF THE INVENTION

The invention relates to a method of detecting one or more sequence variants having a frequency of less than 5% in a nucleic acid population. Sequence variants encompass any sequence differences between two nucleic acid molecules. As such, sequence variants is understood to also refer to, at least, single nucleotide polymorphisms, insertion/deletions (indels), allelic frequencies and nucleotide frequencies - that is, these terms are interchangeable. While different detection techniques are discussed throughout this specification using specific examples, it is understood that the process of the invention can be equally applicable to the detection of any sequence variants. For example, a discussion of a process for detecting SNPs in this disclosure can also be applicable to a process for detecting indels or nucleotide frequencies.

This process of the invention may be used to amplify and sequence specific targeted templates such as those found within, *inter alia,* genomes, tissue samples, heterogeneous cell populations, viral populations or environmental samples. These can include, for example, PCR products, candidate genes, mutational hot spots, evolutionary or medically important variable regions. It could also be used for applications such as whole genome amplification with subsequent whole genome sequencing by using variable or degenerate amplification primers.

To date, the discovery of novel sequence variants, in targeted templates has required either the preparation and sequencing of entire genomes, or prior PCR amplification of a region of interest followed by sequencing of either a pool of PCR product molecules, or by sequencing of single PCR product molecules following their amplification by microbial subcloning. The methods of the invention allow the discovery of novel sequence variants, as well as the assaying of known variants, to be performed at substantially greater depth, with greatly improved sensitivity, speed and lower cost than currently provided by existing technology, while avoiding microbial subcloning.

In this disclosure, a single nucleotide polymorphism (SNP) may be defined as a sequence variation that exists in at least two variants where the least common variant is present in at least 0.001% of the population. It is understood that the methods of the disclosure may be applied to "indels." Therefore, while the instant disclosure makes references to SNP, it is understood that this disclosure is equally applicable if the term "SNP" is substituted with the term "indel" at any location.

As used herein, the term *"indel"* is intended to mean the presence of an insertion or a deletion of one or more nucleotides within a nucleic acid sequence compared to a related nucleic acid sequence. An insertion or a deletion therefore includes the presence or absence of a unique nucleotide or nucleotides in one nucleic acid sequence compared to an otherwise identical nucleic acid sequence at adjacent nucleotide positions. Insertions and deletions can include, for example, a single nucleotide, a few nucleotides or many nucleotides, including 5, 10, 20, 50, 100 or more nucleotides at any particular position compared to a related reference sequence. It is understood that the term also includes more than one insertion or deletion within a nucleic acid sequence compared to a related sequence.

Poisson statistics indicates that the lower limit of detection (i.e., less than one event) for a fully loaded 60mm X 60mm picotiter plate ($2 \times 10^6$ high quality bases, comprised of 200,000 x 100 base reads) is three events with a 95% confidence of detection and five events with a 99% confidence of detection (see Table 1). This scales directly with the number of reads, so the same limits of detection hold for three or five events in 10,000 reads, 1000 reads or 100 reads. Since the actual amount of DNA read is higher than the 200,000, the actual lower limit of detection is expected to at an even lower point due to the increased sensitivity of the assay. For comparison, SNP detection via pyrophosphate based sequencing has been reported for separate allelic states on a tetraploid genome, so long as the least frequent allele was present in 10% or more of the population (Rickert et al., 2002 BioTechniques. 32:592-603). Conventional fluorescent DNA sequencing is even less sensitive, experiencing trouble resolving 50/50 (i.e.; 50 %) heterozygote alleles (Ahmadian et al., 2000 Anal. BioChem. 280:103-110).

Table 1: Probability of detecting zero or one or more events, based on number of events in total population. "*" indicates that probability of failing to detect three events is 5.0%, thus the probability of detecting said event is 95%; similarly, "**" reveals that that probability of detecting one or more events that occur 5 times is 99.3%.

| Copies of Sequence | Percent chance of detecting zero copies | Percent chance of detecting one or more copies |
|---|---|---|
| 1 | 36.8 | 63.2 |
| 2 | 13.5 | 86.5 |
| 3 | 5.0* | 95.0* |
| 4 | 1.8 | 98.2 |
| 5 | 0.7** | 99.3** |
| 6 | 0.2 | 99.8 |
| 7 | 0.1 | 99.9 |
| 8 | 0.0 | 100.0 |
| 9 | 0.0 | 100.0 |
| 10 | 0.0 | 100.0 |

As a result, utilizing an entire 60 x 60 mm picotiter plate to detect a single SNP permits detection of a SNP present in only 0.002% of the population with a 95% confidence or in 0.003% of the population with 99% confidence. Naturally, multiplex analysis is of greater applicability than this depth of detection and Table 2 displays the number of SNPs that can be screened simultaneously on a single picotiter plate, with the minimum allelic frequencies detectable at 95% and 99% confidence.

Table 2

| SNP Classes | Number of Reads | Frequency of SNP in population with 95% confidence | Frequency of SNP in population with 99% confidence |
|---|---|---|---|
| 1 | 200000 | 0.002% | 0.003% |
| 2 | 10000 | 0.030% | 0.050% |
| 5 | 4000 | 0.075% | 0.125% |
| 10 | 2000 | 0.15% | 0.25% |
| 50 | 400 | 0.75% | 1.25% |
| 100 | 200 | 1.50% | 2.5% |
| 150 | 133 | 2.25% | 3.75% |
| 200 | 100 | 3.0% | 5.0% |
| 500 | 40 | 7.5% | 12.5% |
| 1000 | 20 | 15.0% | 25.0% |

One advantage of the invention is that a number of steps, usually associated with sample preparation (e.g., extracting and isolating DNA from tissue for sequencing) may be eliminated or simplified. For example, because of the sensitivity of the method, it is no longer necessary to extract DNA from tissue using traditional technique of grinding tissue and chemical purification. Instead, a small tissue sample of less than one microliter in volume may be boiled and used for the first PCR amplification. The product of this solution amplification is added directly to the emPCR reaction. The methods of the invention therefore reduce the time and effort and product loss (including loss due to human error).

Another advantage of the methods of the invention is that the method is highly amenable to multiplexing. As discussed below, the bipartite primers of the invention allows combining primer sets for multiple genes with identical pyrophosphate sequencing primer sets in a single solution amplification. Alternatively, the product of multiple preparations may be placed in a single emulsion PCR reaction. As a result, the methods of the invention exhibit considerable potential for high throughput applications.

One embodiment of the invention is directed to a method for determining an allelic frequency (including SNP and indel frequency). In the first step, a first population of amplicons is produced by PCR using a first set of primers to amplify a target population of nucleic acids comprising the locus to be analyzed. The locus may comprise a plurality of alleles such as, for example, 2, 4, 10, 15 or 20 or more alleles. The first amplicons may be of any size, such as, for example, between about 50 and about 100 bp, between about 100 bp and about 200 bp, or between about 200 bp and about 1kb, or between about 500 bp and about 5000 bp, or between about 2000 and about 20000 bp. One advantage of the method is that knowledge of the nucleic acid sequence between the two primers is not required.

In the next step, the population of first amplicons is delivered into aqueous microreactors in a water-in-oil emulsion such that a plurality of aqueous microreactors comprises (1) sufficient DNA to initiate an amplification reaction dominated by a single template or amplicon (2) a single bead, and (3) amplification reaction solution containing reagents necessary to perform nucleic acid amplification (See discussion regarding EBCA (Emulsion Based Clonal Amplification) below). We have found that an amplification reaction dominated by a single template or amplicon may be achieved even if two or more templates are present in the microreactor. Therefore, aqueous microreactors comprising more than one template are also described herein. Preferably, each aqueous microreactor has a single copy of DNA template for amplification.

After the delivery step, the first population of amplicons is amplified in the microreactors to form second amplicons. Amplification may be performed, for example, using EBCA (which involves PCR) (described in WO 2004/069849) in a thermocycler to produce second amplicons. After EBCA, the second amplicons can be bound to the beads in the microreactors. The beads, with bound second amplicons are delivered to an array of reaction chambers (e.g., an array of at least 10,000 reaction chambers) on a planar surface. The delivery can be adjusted such that a plurality of the reaction chambers comprise no more than a single bead. This may be accomplished, for example, by using an array where the reaction chambers are sufficiently small to accommodate only a single bead.

A sequencing reaction can be performed simultaneously on the plurality of reaction chambers to determine a plurality of nucleic acid sequences corresponding to said plurality of alleles. Methods of parallel sequencing in parallel using reaction chambers are disclosed in another section above and in the Examples. Following sequencing, the allelic frequency, for at least two alleles, may be determined by analyzing the sequences from the target population of nucleic acids. As an example, if 10000 sequences are determined and 9900 sequences read "aaa" while 100 sequences read "aag," the "aaa" allele may be said to have a frequency of about 99% while the "aag" allele would have a frequency of about 1%. This is described in more detail in the description below and in the Examples.

One advantage of the invention's methods is that it allows a higher level of sensitivity than previously achieved. If a picotiter plate is used, the methods of the invention can sequence over 100,000 or over 300,000 different copies of an allele per picotiter plate. The sensitivity of detection should allow detection of low abundance alleles which may represent about 1% or less of the allelic variants. Another advantage of the invention's methods is that the sequencing reaction also provides the sequence of the analyzed region. That is, it is not necessary to have prior knowledge of the sequence of the locus being analyzed.

In a preferred embodiment, the methods of the invention may detect an allelic frequency which is less than about 5%, or less than about 2%. In a more preferred embodiment, the method may detect allelic frequencies of less than about 1%, such as less than about 0.5%, less than about 0.2%, or less than about 0.02%. Typical ranges of detection sensitivity may be between about 0.01% and about 100%, between about 0.01% and about 50%, between about 0.01% and about 10% such as between about 0.1% and about 5%.

The target population of nucleic acids may be from a number of sources. For example, the source may be a tissue or body fluid from an organism. The organism may be any organism including, but not limited to, mammals. The mammals may be a human or a commercially valuable livestock such as cows, sheep, pigs, goats, rabbits, and the like. The method of the invention would allow analysis of tissue and fluid samples of plants. While all plants may be analyzed by the methods of the invention, preferred plants for the methods of the invention include commercially valuable crops species including monocots and dicots. In one preferred embodiment, the target population of nucleic acids may be derived from a grain or food product to determine the original and distribution of genotypes, alleles, or species that make up the grain or food product. Such crops include, for example, maize, sweet corn, squash, melon, cucumber, sugarbeet, sunflower, rice, cotton, canola, sweet potato, bean, cowpea, tobacco, soybean, alfalfa, wheat, or the like.

Nucleic acid samples may be collected from multiple organisms. For example, allelic frequency of a population of 1000 individuals may be performed in one experiment analyzing a mixed DNA sample from 1000 individuals. Naturally, for a mixed DNA sample to be representative of the allelic frequency of a population, each member of the population (each individual) must contribute the same (or approximately the same) amount of nucleic acid (same number of copies of an allele) to the pooled sample. For example, in an analysis of genomic allelic frequency, each individual may contribute the DNA from approximately $1.0 \times 10^6$ cells to a pooled DNA sample.

In another embodiment of the invention, the polymorphism in a single individual may be determined. That is the target nucleic acid may be isolated from a single individual. For example, pooled nucleic acids from multiple tissue sample of an individual may be examined for polymorphisms and nucleotide frequencies. This may be useful, for example, for determining polymorphism in a tumor, or a tissue suspected to contain a tumor, of an individual. The method of the invention may be used, for example, to determine the frequency of an activated oncogene in a tissue sample (or pooled DNA from multiple tissue sample) of an individual. In this example, an allelic frequency of 50% or more of activated oncogenes may indicate that the tumor is monoclonal. The presence of less than 50% of an activated oncogene may indicate that the tumor is polyclonal, or that the tissue sample contains a combination of tumor tissue and normal (non-tumor) tissue. Furthermore, in a biopsy of a suspect tissue, the presence of, for example, 1% of an activated oncogene may indicate the presence of an emerging tumor, or the presence of a malignant tumor infiltration. In addition, the presence of a fraction of tumor cells having a drug resistance mutation, in an otherwise drug sensitive tumor, may predict a relapse of the patient with an entirely drug resistant tumor. Such prognostic information will be invaluable in cancer therapy and research.

The target population of nucleic acids may be any nucleic acid including, DNA, RNA and various forms of such DNA and RNAs such as, but not limited to plasmids, cosmids, DNA viral genomes, RNA viral genome, bacterial genomes, fungal genomes, protozoal genomes, mitochondrial DNA, mammalian genomes, and plant genomes. The nucleic acid may be isolated from a tissue sample or from an *in vitro* culture. Genomic DNA can be isolated from a tissue sample, a whole organism, or a sample of cells. If desired, the target population of nucleic acid may be normalized such that it contains an equal amount of alleles from each individual that contributed to the population.

One advantage of the invention is that the genomic DNA may be used directly without further processing. However, in a preferred embodiment, the genomic DNA may be substantially free of proteins that interfere with PCR or hybridization processes, and are also substantially free of proteins that damage DNA, such as nucleases. Preferably, the isolated genomes are also free of non-protein inhibitors of polymerase function (e.g. heavy metals) and non-protein inhibitors of hybridization which would interfere with a PCR. Proteins may be removed from the isolated genomes by many methods known in the art. For instance, proteins may be removed using a protease, such as proteinase K or pronase, by using a strong detergent such as sodium dodecyl sulfate (SDS) or sodium lauryl sarcosinate (SLS) to lyse the cells from which the isolated genomes are obtained, or both. Lysed cells may be extracted with phenol and chloroform to produce an aqueous phase containing nucleic acid, including the isolated genomes, which can be precipitated with ethanol.

The target population of nucleic acid may be derived from sources with unknown origins of DNA such as soil samples, food samples and the like. For example, the sequencing of an allele found in a pathogen in a nucleic acid sample from a food sample would allow the determination the presence of pathogen contamination in the food. Furthermore, the methods of the invention would allow determination of the distribution of a pathogenic allele in the food. For example, the methods of the invention can determine the strain (species) or distribution of strains (species) of a particular organism (e.g., bacteria, virus, pathogens) in an environmental sample such as a soil sample (See, Example 5) or a seawater sample.

One advantage of the method provided herein is that *a priori* knowledge of mutations or sequence variants in a nucleic acid or polynucleotide population is not required for the method. Because the method is based on nucleic acid sequencing, all mutations in one location would be detected. Furthermore, no microbial cloning is required for the sequencing. A DNA sample can be amplified and sequenced in vitro in a series of steps without the need for cloning, subcloning, and culturing of the cloned DNA.

The methods of the invention may be used, for example, for detection and quantification of variants in viral samples. These viral samples may include, for example, an HIV viral isolate. Other applications of the method include population studies of sequence variants. DNA samples may be collected from a population of organisms and combined and analyzed in one experiment to determine allelic frequencies. The populations of organisms may include, for example, a population of humans, a population of livestock, a population of grain from a harvest and the like. Other uses include detection and quantification of somatic mutations in tumor biopsies (e.g. lung and colorectal cancer) or from biopsies comprising a mixed population of tumor and normal cells. The methods of the invention may also be used for high confidence re-sequencing of clinically relevant susceptibility genes (e.g. breast, ovarian, colorectal and pancreatic cancer, melanoma).

Another use for the invention involves identification of polymorphisms associated with a plurality of distinct genomes. The distinct genomes may be isolated from populations which are related by some phenotypic characteristic, familial origin, physical proximity, race, class, etc. In other cases, the genomes are selected at random from populations such that they have no relation to one another other than being selected from the same population. It is herein disclosed that the method can be performed to determine the genotype (e.g. SNP content) of subjects having a specific phenotypic characteristic, such as a genetic disease or other trait.

The methods of the invention may also be used to characterize the genetic makeup of a tumor by testing for loss of heterozygosity or to determine the allelic frequency of a particular SNP. Additionally, the methods may be used to generate a genomic classification code for a genome by identifying the presence or absence of each of a panel of SNPs in the genome and to determine the allelic frequency of the SNPs. Each of these uses is discussed in more detail herein.

A use of the methods described herein comprises a high throughput method of genotyping. "Genotyping" is the process of identifying the presence or absence of specific genomic sequences within genomic DNA. Distinct genomes may be isolated from individuals of populations which are related by some phenotypic characteristic, by familial origin, by physical proximity, by race, by class, etc. in order to identify polymorphisms (e.g. ones associated with a plurality of distinct genomes) which are correlated with the phenotype family, location, race, class, etc. Alternatively, distinct genomes may be isolated at random from populations such that they have no relation to one another other than their origin in the population. Identification of polymorphisms in such genomes indicates the presence or absence of the polymorphisms in the population as a whole, but not necessarily correlated with a particular phenotype. Since a genome may span a long region of DNA and may involve multiple chromosomes, a method for detecting a genotype would need to analyze a plurality of sequence variants at multiple locations to detect a genotype at a reliability of 99.99%.

Although genotyping is often used to identify a polymorphism associated with a particular phenotypic trait, this correlation is not necessary. Genotyping only requires that a polymorphism, which may or may not reside in a coding region, is present. When genotyping is used to identify a phenotypic characteristic, it is presumed that the polymorphism affects the phenotypic trait being characterized. A phenotype may be desirable, detrimental, or, in some cases, neutral. Polymorphisms identified according to the methods of the invention can contribute to a phenotype. Some polymorphisms occur within a protein coding sequence and thus can affect the protein structure, thereby causing or contributing to an observed phenotype. Other polymorphisms occur outside of the protein coding sequence but affect the expression of the gene. Still other polymorphisms merely occur near genes of interest and are useful as markers of that gene. A single polymorphism can cause or contribute to more than one phenotypic characteristic and, likewise, a single phenotypic characteristic may be due to more than one polymorphism. In general, multiple polymorphisms occurring within the same haplotype of a given gene correlate with the same phenotype. Additionally, whether an individual is heterozygous or homozygous for a particular polymorphism can affect the presence or absence of a particular phenotypic trait.

Phenotypic correlation can be performed by identifying an experimental population of subjects exhibiting a phenotypic characteristic and a control population which do not exhibit that phenotypic characteristic. Polymorphisms which occur within the experimental population of subjects sharing a phenotypic characteristic and which do not occur in the control population are said to be polymorphisms which are correlated with a phenotypic trait. Once a polymorphism has been identified as being correlated with a phenotypic trait, genomes of subjects which have potential to develop a phenotypic trait or characteristic can be screened to determine occurrence or non-occurrence of the polymorphism in the subjects' genomes in order to establish whether those subjects are likely to eventually develop the phenotypic characteristic. These types of analyses are may be performed on subjects at risk of developing a particular disorder such as Huntington's disease or breast cancer.

The methods herein described may be used for associating a phenotypic trait with a SNP. A phenotypic trait encompasses any type of genetic disease, condition, or characteristic, the presence or absence of which can be positively determined in a subject. Phenotypic traits that are genetic diseases or conditions include multifactorial diseases of which a component may be genetic (e.g. owing to occurrence in the subject of a SNP), and predisposition to such diseases. These diseases include such as, but not limited to, asthma, cancer, autoimmune diseases, inflammation, blindness, ulcers, heart or cardiovascular diseases, nervous system disorders, and susceptibility to infection by pathogenic microorganisms or viruses. Autoimmune diseases include, but are not limited to, rheumatoid arthritis, multiple sclerosis, diabetes, systemic lupus, erythematosus and Graves disease. Cancers include, but are not limited to, cancers of the bladder, brain, breast, colon, esophagus, kidney, hematopoietic system e.g. leukemia, liver, lung, oral cavity, ovary, pancreas, prostate, skin, stomach, and uterus. A phenotypic trait may also include susceptibility to drug or other therapeutic treatments, appearance, height, color (e.g. of flowering plants), strength, speed (e.g. of race horses), hair color, etc. Many examples of phenotypic traits associated with genetic variation have been described, see e.g., U.S. Pat. No. 5,908,978 (which identifies association of disease resistance in certain species of plants associated with genetic variations) and U.S. Pat. No. 5,942,392 (which describes genetic markers associated with development of Alzheimer's disease).

Identification of associations between genetic variations (e.g. occurrence of SNPs) and phenotypic traits is useful for many purposes. For example, identification of a correlation between the presence of a SNP allele in a subject and the ultimate development by the subject of a disease is particularly useful for administering early treatments, or instituting lifestyle changes (e.g., reducing cholesterol or fatty foods in order to avoid cardiovascular disease in subjects having a greater-than-normal predisposition to such disease), or closely monitoring a patient for development of cancer or other disease. It may also be useful in prenatal screening to identify whether a fetus is afflicted with or is predisposed to develop a serious disease. Additionally, this type of information is useful for screening animals or plants bred for the purpose of enhancing or exhibiting of desired characteristics.

One method for determining an SNP or a plurality of SNPs associated with a plurality of genomes is screening for the presence or absence of a SNP in a plurality of genomic samples derived from organisms with the trait. In order to determine which SNPs are related to a particular phenotypic trait, genomic samples are isolated from a group of individuals which exhibit the particular phenotypic trait, and the samples are analyzed for the presence of common SNPs. The genomic sample obtained from each individual may be combined to form a pooled genomic sample. Then the methods of the invention are used to determine an allelic frequency for each SNP. The pooled genomic sample is screened using panels of SNPs in a high throughput method to determine whether the presence or absence of a particular SNP (allele) is associated with the phenotype. In some cases, it may be possible to predict the likelihood that a particular subject will exhibit the related phenotype. If a particular polymorphic allele is present in 30% of individuals who develop Alzheimer's disease but only in 1% of the population, then an individual having that allele has a higher likelihood of developing Alzheimer's disease. The likelihood can also depend on several factors such as whether individuals not afflicted with Alzheimer's disease have this allele and whether other factors are associated with the development of Alzheimer's disease. This type of analysis can be useful for determining a probability that a particular phenotype will be exhibited. In order to increase the predictive ability of this type of analysis, multiple SNPs associated with a particular phenotype can be analyzed and the correlation values identified.

It is also possible to identify SNPs which segregate with a particular disease. Multiple polymorphic sites may be detected and examined to identify a physical linkage between them or between a marker (SNP) and a phenotype. This may be used to map a genetic locus linked to or associated with a phenotypic trait to a chromosomal position and thereby revealing one or more genes associated with the phenotypic trait. If two polymorphic sites segregate randomly, then they are either on separate chromosomes or are distant enough with respect to one another on the same chromosome that they do not co-segregate. If two sites co-segregate with significant frequency, then they are linked to one another on the same chromosome. These types of linkage analyses can be useful for developing genetic maps which may define regions of the genome important for a phenotype - including a disease genotype.

Linkage analysis may be performed on family members who exhibit high rates of a particular phenotype or a particular disease. Biological samples can be isolated from family members exhibiting a phenotypic trait, as well as from subjects which do not exhibit the phenotypic trait. These samples can each be used to generate individual SNPs allelic frequencies. The data can be analyzed to determine whether the various SNPs are associated with the phenotypic trait and whether or not any SNPs segregate with the phenotypic trait.

Methods for analyzing linkage data have been described in many references, including Thompson & Thompson, Genetics in Medicine (5th edition), W.B. Saunders Co., Philadelphia, 1991; and Strachan, "Mapping the Human Genome" in the Human Genome (Bios Scientific Publishers Ltd., Oxford) chapter 4, and summarized in PCT published patent application WO98/18967 by Affymetrix, Inc. Linkage analysis involving by calculating log of the odds values (LOD values) reveals the likelihood of linkage between a marker and a genetic locus at a recombination fraction, compared to the value when the marker and genetic locus are not linked. The recombination fraction indicates the likelihood that markers are linked. Computer programs and mathematical tables have been developed for calculating LOD scores of different recombination fraction values and determining the recombination fraction based on a particular LOD score, respectively. See e.g., Lathrop, PNAS, USA 81, 3443-3446 (1984); Smith et al., Mathematical Tables for Research Workers in Human Genetics (Churchill, London, 1961); Smith, Ann. Hum. Genet. 32, 127-1500 (1968). Use of LOD values for genetic mapping of phenotypic traits is described in PCT published patent application WO98/18967 by Affymetrix, Inc. In general, a positive LOD score value indicates that two genetic loci are linked and a LOD score of +3 or greater is strong evidence that two loci are linked. A negative value suggests that the linkage is less likely.

The methods herein described are also useful for assessing loss of heterozygosity in a tumor. Loss of heterozygosity in a tumor is useful for determining the status of the tumor, such as whether the tumor is an aggressive, metastatic tumor. The method can be performed by isolating genomic DNA from tumor sample obtained from a plurality of subjects having tumors of the same type, as well as from normal (i.e., non-cancerous) tissue obtained from the same subjects. These genomic DNA samples can be used in the SNP detection method of the invention. The absence of a SNP allele from the tumor compared to the SNP alleles generated from normal tissue indicates whether loss of heterozygosity has occurred. If a SNP allele is associated with a metastatic state of a cancer, the absence of the SNP allele can be compared to its presence or absence in a non-metastatic tumor sample or a normal tissue sample. A database of SNPs which occur in normal and tumor tissues can be generated and an occurrence of SNPs in a patient's sample can be compared with the database for diagnostic or prognostic purposes.

It is useful to be able to differentiate non-metastatic primary tumors from metastatic tumors, because metastasis is a major cause of treatment failure in cancer patients. If metastasis can be detected early, it can be treated aggressively in order to slow the progression of the disease. Metastasis is a complex process involving detachment of cells from a primary tumor, movement of the cells through the circulation, and eventual colonization of tumor cells at local or distant tissue sites. Additionally, it is desirable to be able to detect a predisposition for development of a particular cancer such that monitoring and early treatment may be initiated. Many cancers and tumors are associated with genetic alterations.

Solid tumors progress from tumorigenesis through a metastatic stage and into a stage at which several genetic aberrations can occur. e.g., Smith et al., Breast Cancer Res. Terat., 18 Suppl. 1, S5-14, 1991. Genetic aberrations are believed to alter the tumor such that it can progress to the next stage, i.e., by conferring proliferative advantages, the ability to develop drug resistance or enhanced angiogenesis, proteolysis, or metastatic capacity. These genetic aberrations are referred to as "loss of heterozygosity." Loss of heterozygosity can be caused by a deletion or recombination resulting in a genetic mutation which plays a role in tumor progression. Loss of heterozygosity for tumor suppressor genes is believed to play a role in tumor progression. For instance, it is believed that mutations in the retinoblastoma tumor suppressor gene located in chromosome 13q14 causes progression of retinoblastomas, osteosarcomas, small cell lung cancer, and breast cancer. Likewise, the short arm of chromosome 3 has been shown to be associated with cancer such as small cell lung cancer, renal cancer and ovarian cancers. For instance, ulcerative colitis is a disease which is associated with increased risk of cancer presumably involving a multistep progression involving accumulated genetic changes (U.S. Pat. No. 5,814,444). It has been shown that patients afflicted with long duration ulcerative colitis exhibit an increased risk of cancer, and that one early marker is loss of heterozygosity of a region of the distal short arm of chromosome 8. This region is the site of a putative tumor suppressor gene that may also be implicated in prostate and breast cancer. Loss of heterozygosity can easily be detected by performing the methods herein described routinely on patients afflicted with ulcerative colitis. Similar analyses can be performed using samples obtained from other tumors known or believed to be associated with loss of heterozygosity. This is particularly advantageous for studying loss of heterozygosity because thousands of tumor samples can be screened at one time.

The disclosure includes, in part, methods for processing nucleic acids to determine an allelic frequency. One of these methods may be broadly defined in the following three steps: (1) Sample preparation - preparation of the first amplicons; (2) bead emulsion PCR - preparation of the second amplicons. (3) sequencing by synthesis - determining multiple sequences from the second amplicons to determine an allelic frequency. Each of these steps is described in more detail below and in the Example section.

## 1. NUCLEIC ACID TEMPLATE PREPARATION

### Nucleic Acid Templates

The template nucleic acid can be constructed from any source of nucleic acid, e.g., any cell, tissue, or organism, and can be generated by any art-recognized method. Alternatively, template libraries can be made by generating a complementary DNA (cDNA) library from RNA, e.g., messenger RNA (mRNA). Methods of sample preparation may be found in copending U.S. Patent Application Serial No. 10/767,779 and PCT application PCT/US04/02570 and is also published in WO/04070007.

The methods of the present invention comprise the selective amplification of a polynucleotide region of interest from a population of first polynucleotide molecules. The amplification will result in a population of second polynucleotide molecules that are derived from a plurality of first molecules comprising the region of interest. Even though each of the first molecules amplified comprises the region of interest, it will be appreciated that one or more sequence variations may exist between the first molecules within the region of interest. The number of individual first molecules in the population thus amplified may range from 2 to several billion, advantageously, more than about 100, more than about 1000, more than about 10,000, more than about 100,000, more than about 1 million, or more than about 1 billion molecules.

Selective amplification means that the amplification is directed at a region of interest and thus preferentially or specifically amplifies that region of interest. Ideally, only the region of interest will experience amplification. However, the skilled artisan will appreciate that substantial non-specific amplification of other regions may also occur, as is frequently observed in nucleic acid amplification reactions. Such non-specific reaction products may be avoided by optimization of the reaction conditions, such as by modifications of the temperature, primer design and concentration, the concentration of buffer components and nucleotides, and the like. The skilled artisan will be familiar with strategies for the optimization of amplification reactions, including the use of nested primers for improvement of amplification specificity. Alternatively, any non-specific amplification products may be separated from the desired products, for example by size selection through gel electrophoresis or chromatographic techniques. Depending on the degree of the non-specific amplification and the specific experimental design, removal of non-specific products may not be necessary at all.

The selective amplification reaction may be performed by a number of methods known in the art, including isothermal methods and methods requiring thermocycling. For example, a thermocycling method readily known to those in the art is the polymerase chain reaction (PCR). An example of an isothermal method for selective amplification is the loop-mediated isothermal amplification (LAMP) described by Notomi et al., Nucleic Acids Res. 2000;28(12):E63. LAMP employs the self-recurring strand displacement DNA synthesis primed by a specifically designed set of target specific primers. The size of the polynucleotide region of interest, i.e. its length, will range between about 20 and about 40,000 nucleotides, such as between about 50 and about 10,000 nucleotides, between about 80 and about 1000 nucleotides, or between about 100 and about 500 nucleotides. A length between about 50 and about 2000 nucleotides is preferred.

The amplification product may be in the form of a single stranded or double stranded polynucleotide, or both. These and other methods of DNA amplification are described in: DNA Amplification: Current Technologies and Applications, V. Demidov and N. Broude, eds., Horizon Bioscience, 2004. Combinations of any such different amplification methods are also contemplated.

Regardless of the method used, the selective amplification will result in the synthesis of a population(s) of second polynucleotide molecules. The number of individual second polynucleotide molecules in the population thus amplified may range from 2 to several billion, advantageously, more than about 100, more than about 1000, more than about 10000, more than about 100,000, more than about 1 million, or more than about 1 billion molecules. The amplified polynucleotide region may range from 2 to several billion nucleotides, advantageously comprising at least about 25, at least about 50, at least about 150, at least about 300, at least about 500, at least about 1000, at least about 5000, or at least about 10,000 nucleotides.

The selective amplification may also be targeted to a plurality of regions of interest, either in separate reactions, or in a single reaction (i.e. multiplexing). If such a plurality of regions was amplified separately, the amplification products may be combined (pooled) at any point prior to the sequence determination step.

One preferred method of nucleic acid template preparation is to perform PCR on a sample to amplify a region containing the (known or suspected) allele or alleles of interest. The PCR technique can be applied to any nucleic acid sample (DNA, RNA, cDNA) using oligonucleotide primers spaced apart from each other. The primers are complementary to opposite strands of a double stranded DNA molecule and are typically separated by from about 50 to 2000 nucleotides, or more. However, the PCR amplification of regions as large as 35000 bases is possible by use of proofreading DNA polymerases (Barnes, W. M. (1994) Proc. Natl. Acad. Sci. USA 91:2216). The PCR method is described in a number of publications, including Saiki et al., Science (1985) 230:1350-1354; Saiki et al., Nature (1986) 324:163-166; and Scharf et al., Science (1986) 233:1076- 1078. Also see U.S. Pat. Nos. 4,683,194; 4,683,195; and 4,683,202. Additional methods for PCR amplification are described in: PCR Technology: Principles and Applications for DNA Amplification ed. HA Erlich, Freeman Press, New York, N.Y. (1992); PCR Protocols: A Guide to Methods and Applications, eds. Innis, Gelfland, Snisky, and White, Academic Press, San Diego, Calif. (1990); Mattila et al. (1991) Nucleic Acids Res. 19: 4967; Eckert, K. A. and Kunkel, T. A. (1991) PCR Methods and Applications 1: 17, and; PCR, eds. McPherson, Quirkes, and Taylor, IRL Press, Oxford.

## 2. NUCLEIC ACID TEMPLATE AMPLIFICATION

The population(s) of second polynucleotide molecules can then be subjected to sequence analysis, whereby single second polynucleotide molecules are separately sequenced.

Optionally however, prior to sequence analysis, the single second polynucleotide molecules are subjected to a second round of *in vitro* amplification, resulting in the synthesis of population(s) of third polynucleotide molecules. This second round of amplification can occur by any one of several methods known in the art that allow a third molecule population derived from each second molecule to remain separated from the third molecule populations that result from the other second molecules. This type of amplification is commonly referred to as clonal amplification. "Clonal" , as used herein, means being comprised of a plurality of identical molecules, or copies, such as, for example, being comprised of a plurality of identical nucleic acid molecules amplified from a single ancestor nucleic acid molecule. Specifically, each population(s) is clonal in that it represents a single second polynucleotide molecule in the subsequent sequence determination.

In one embodiment, the second round of amplification may be performed on a solid or semi-solid support, such as, for example, by an amplification method known as a bridge amplification, as described in U.S. Patent Application Publication No. 2005/0100900, in U.S. Patent Application Publication No. 2003/0022207, and in U.S. Patent Application Publication No. 2004/0096853. Accordingly, the second polynucleotide molecules may be annealed to appropriate oligonucleotide primer molecules, which are immobilized on a solid support. The primer may then be extended and the molecule and the primer may be separated from one another. The extended primer may then be annealed to another immobilized primer (thus forming a "bridge") and the other primer may be extended. Both extended primers may then be separated from one another and may be used to provided further extended primers. The process may be repeated to provide amplified, immobilized population(s) of third polynucleotide molecules. If the initial annealing of the second polynucleotide molecules was performed such that the annealed molecules are at sufficient distance from each other, the population(s) of third polynucleotides will tend to remain separated from each other in the form of colonies and will therefore be clonal. Thus, even though the colonies may be in close proximity to one another on a single solid or semi-support, under appropriate starting conditions the majority of colonies will nonetheless be distinct and represent clonal amplification products. These colonies comprising bridge amplification products may then be subjected to nucleotide sequence analysis.

In another embodiment, the second round of amplification may be performed by amplification in an emulsion (WO 2004/069849 and WO 2005/073410). The emulsion may contain millions of individual reactions. The emulsion may contain microparticles with which the amplification products become associated in a clonal fashion.

In yet another embodiment, the second round of amplification may be performed on a semi-solid support, for example by the polony technology described in US Patent Nos. 6,432,360; 6,485,944; and 6,511,803. For example, oligonucleotide primers are immobilized on a semi-solid support, template nucleic acids are seeded onto the semi-solid support and hybridized to the primers, which are extended using DNA polymerase and deoxynucleotide triphosphates, then denatured. Several rounds of annealing, extension and denaturation leads to clonal amplification *in situ* on the semi-solid support. The amplification products are spatially restricted to the immediate vicinity of the template molecule from which they are derived. This results in the creation of PCR colonies, known in the art as polonies. The polynucleotide sequence of the nucleic acid molecules in each polony can then be determined by a number of methods known in the art, including sequencing-by-synthesis methods, for example as described by Mitra et al. (2003) Analyt. Biochem. 320: 55-65.

In a preferred embodiment, the second round of amplification may be performed by a novel amplification system, herein termed EBCA (Emulsion Based Clonal Amplification or bead emulsion amplification) is used to perform this second amplification. EBCA (WO 2004/069849 and WO 2005/073410) is performed by attaching a template nucleic acid (e.g., DNA) to be amplified to a solid support, preferably in the form of a generally spherical bead. A library of single stranded template DNA prepared according to the sample preparation methods of this invention is an example of one suitable source of the starting nucleic acid template library to be attached to a bead for use in this amplification method.

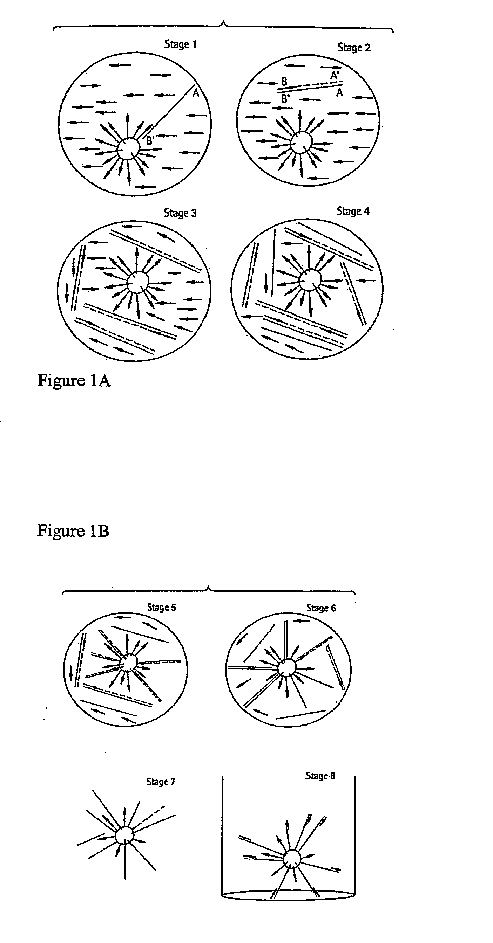

The bead is linked to a large number of a single primer species (i.e., primer B in Figure 1) that is complementary to a region of the template DNA. Template DNA annealed to the bead bound primer. The beads are suspended in aqueous reaction mixture and then encapsulated in a water-in-oil emulsion. The emulsion is composed of discrete aqueous phase microdroplets, approximately 60 to 200 μm in diameter, enclosed by a thermostable oil phase. Each microdroplet contains, preferably, amplification reaction solution (i.e., the reagents necessary for nucleic acid amplification). An example of an amplification would be a PCR reaction mix (polymerase, salts, dNTPs) and a pair of PCR primers (primer A and primer B). See, Figure 1A. A subset of the microdroplet population also contains the DNA bead comprising the DNA template. This subset of microdroplet is the basis for the amplification. The microcapsules that are not within this subset have no template DNA and will not participate in amplification. The amplification technique is PCR and the PCR primers may be present in a 8:1 or 16:1 ratio (i.e., 8 or 16 of one primer to 1 of the second primer) to perform asymmetric PCR.

In this overview, the DNA is annealed to an oligonucleotide (primer B) which is immobilized to a bead. During thermocycling (Figure 1B), the bond between the single stranded DNA template and the immobilized B primer on the bead is broken, releasing the template into the surrounding microencapsulated solution. The amplification solution, in this case, the PCR solution, contains addition solution phase primer A and primer B. Solution phase B primers readily bind to the complementary b' region of the template as binding kinetics are more rapid for solution phase primers than for immobilized primers. In early phase PCR, both A and B strands amplify equally well (Figure 1C).

By midphase PCR (*i.e.*, between cycles 10 and 30) the B primers are depleted, halting exponential amplification. The reaction then enters asymmetric amplification and the amplicon population becomes dominated by A strands (Figure 1D). In late phase PCR (Figure 1E), after 30 to 40 cycles, asymmetric amplification increases the concentration of A strands in solution. Excess A strands begin to anneal to bead immobilized B primers. Thermostable polymerases then utilize the A strand as a template to synthesize an immobilized, bead bound B strand of the amplicon.

In final phase PCR (Figure 1F), continued thermal cycling forces additional annealing to bead bound primers. Solution phase amplification may be minimal at this stage but concentration of immobilized B strands increase. Then, the emulsion is broken and the immobilized product is rendered single stranded by denaturing (by heat, pH etc.) which removes the complimentary A strand. The A primers are annealed to the A' region of immobilized strand, and immobilized strand is loaded with sequencing enzymes, and any necessary accessory proteins. The beads are then sequenced using recognized pyrophosphate techniques (described, e.g., in U.S. Patent Nos. 6,274,320, 6,258,568 and 6,210,891.

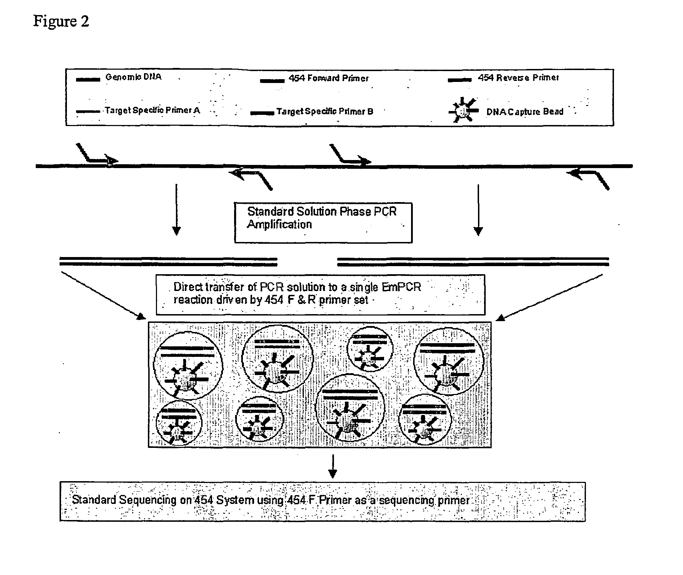

The primers used for amplification are bipartite - comprising a 5' section and a 3' section. The 3' section of the primer contains target specific sequence (see Figure 2) and performed the function of PCR primers. The 5' section of the primer comprises sequences which are useful for the sequencing method or the immobilization method. For example, in Figure 2, the 5' section of the two primers used for amplification contains sequences (labeled 454 forward and 454 reverse) which are complementary to primers on a bead or a sequencing primer. That is, the 5' section, containing the forward or reverse sequence, allows the amplicons to attach to beads that contain immobilized oligos which are complementary to the forward or reverse sequence. Furthermore, sequencing reaction may be initiated using sequencing primers which are complementary to the forward and reverse primer sequences. Thus one set of beads comprising sequences complementary to the 5' section of the bipartite primer may be used on all reactions. Similarly, one set of sequencing primers comprising sequences complementary to the 5' section of the bipartite primer may be used to sequence any amplicons made using the bipartite primer. In the most preferred embodiment, all bipartite primer sets used for amplification would have the same set of 5' sections such as the 454 forward primer and 454 reverse primer shown in Figure 2. In this case, all amplicons may be analyzed using standard beads coated with oligos complementary to the 5' section. The same oligos (immobilized on beads or not immobilized) may be used as sequencing oligos.

## Breaking the Emulsion and Bead Recovery

Following amplification of the template, the emulsion is "broken" (also referred to as "demulsification" in the art). There are many methods of breaking an emulsion (see, e.g., U.S. Patent No. 5,989,892 and references cited therein) and one of skill in the art would be able to select the proper method. One preferred method of breaking the emulsion is described in detail in the Example section.

After the emulsion is broken, the amplified template-containing beads may then be resuspended in aqueous solution for use, for example, in a sequencing reaction according to known technologies. (See, Sanger, F. et al., Proc. Natl. Acad. Sci. U.S.A. 75, 5463-5467 (1977); Maxam, A. M. & Gilbert, W. Proc Natl Acad Sci USA 74, 560-564 (1977); Ronaghi, M. et al., Science 281, 363, 365 (1998); Lysov, I. et al., Dokl Akad Nauk SSSR 303, 1508-1511 (1988); Bains W. & Smith G. C. J.TheorBiol 135, 303-307(1988); Drnanac, R. et al., Genomics 4, 114-128 (1989); Khrapko, K. R. et al., FEBS Lett 256. 118-122 (1989); Pevzner P. A. J Biomol Struct Dyn 7, 63-73 (1989); Southern, E. M. et al., Genomics 13, 1008-1017 (1992).) If the beads are to be used in a pyrophosphate-based sequencing reaction (described, e.g., in U.S. Patent Nos. 6,274,320, 6258,568 and 6,210,891, then it is necessary to remove the second strand of the PCR product and anneal a sequencing primer to the single stranded template that is bound to the bead.

At this point, the amplified DNA on the bead may be sequenced either directly on the bead or in a different reaction vessel. In an embodiment of the present invention, the DNA is sequenced directly on the bead by transferring the bead to a reaction vessel and subjecting the DNA to a sequencing reaction (e.g., pyrophosphate or Sanger sequencing). Alternatively, the beads may be isolated and the DNA may be removed from each bead and sequenced. In either case, the sequencing steps may be performed on each individual bead.

## 3. METHODS OF SEQUENCING NUCLEIC ACIDS

According to the invention, each of a plurality or population of second polynucleotide molecules, or optionally each of a plurality or population of third polynucleotide molecules, are subjected to nucleotide sequence analysis. The sequence of the second (and optionally third) polynucleotide molecules is determined by the methods of the invention, ranging from 2 to several billion, advantageously from more than about 100, more than about 1000, more than about 10,000, more than about 100,000, more than about 1 million, or more than about 1 billion. The sequence can comprise at least two consecutive nucleotides, preferably at least about 5, at least about 25, at least about 50, at least about 100, at least about 150, at least about 200, at least about 300, at least about 500, at least about 1000, at least about 5000, at least about 10,000, or at least about 100,000 consecutive nucleotides and are determined from each of the second (or optionally third) polynucleotide molecules.

The skilled artisan will be familiar with several methods for sequencing of polynucleotides. These include, but are not limited to, Sanger sequencing (also referred to as dideoxy sequencing) and various sequencing-by-synthesis (SBS) methods as reviewed by Metzger (Metzger ML 2005, Genome Research 1767), sequencing by hybridization, by ligation (for example, WO 2005/021786), by degradation (for example, U.S. Patent Nos. 5,622,824 and 6,140,053) and nanopore sequencing.

Any methods of polynucleotide amplification and sequencing known in the art can be used according to the present invention, as long as the chosen approach results in the sequence determination of single polynucleotide molecules, or optionally the sequence determination of clonal polynucleotide populations derived by amplification from said single polynucleotide molecules. Any amplification occurs *in vitro,* as opposed to by microbial cloning.

In certain embodiments, polynucleotide sequencing is achieved by any of a group of methods referred to as sequencing-by-synthesis (SBS). SBS refers to methods for determining the identity of one or more nucleotides in a polynucleotide or in a population of polynucleotides, wherein the methods comprise the stepwise synthesis of a single strand of polynucleotide complementary to the template polynucleotide whose nucleotide sequence is to be determined. An oligonucleotide primer is designed to anneal to a predetermined, complementary position of the sample template molecule. The primer/template complex is presented with a nucleotide in the presence of a nucleic acid polymerase enzyme. If the nucleotide is complementary to the position on the sample template molecule that is directly adjacent to the 3' end of the oligonucleotide primer, then the polymerase will extend the primer with the nucleotide. Alternatively, the primer/template complex is presented with all nucleotides of interest (typically A, G, C, and T) at once, and the nucleotide that is complementary to the position on the sample template molecule directly adjacent to the 3' end of the oligonucleotide primer is incorporated. In either scenario, the nucleotides may be chemically blocked (such as at the 3'-O position) to prevent further extension, and need to be deblocked prior to the next round of synthesis. Any incorporation of the nucleotide can be detected by a variety of methods known in the art, e.g. by detecting the release of pyrophosphate (PPi) (U.S. Patent Nos. 6,210,891; 6,258,568; and 6,828,100) via chemiluminescence, or by use of detectable labels bound to the nucleotides. Detectable labels include mass tags (for example, U.S. Patent Nos. 5,622,824 and 6,140,053) and fluorescent or chemiluminescent labels. The detectable label is bound directly or indirectly to the nucleotides. In the case of fluorescent labels, the label may be excited directly by an external light stimulus, or indirectly by emission from a fluorescent (FRET) or luminescent (LRET) donor (U.S. Patent No. 6,982,146). After detection of the detectable label, the label has to be inactivated, or separated from the reaction, so that it will not interfere or mix with the signal from a subsequent label. Label separation can be achieved, for example, by chemical cleavage (for example U.S. Patent Application Publication No. 2003/0124594) or photocleavage. Label inactivation can be achieved, for example, by photobleaching. According to the invention, any SBS method known in the art may be used in the sequencing of the second polynucleotides, or of the population(s) of third polynucleotides.

According to the invention, polynucleotide sequencing can also be achieved by a nanopore-based method. The underlying principle of nanopore sequencing is that a single-stranded DNA or RNA molecule can be electrophoretically driven through a nano-scale pore in such a way that the molecule traverses the pore in a strict linear fashion. Because a translocating molecule partially obstruct or blocks the nanopore, it alters the pore's electrical properties. This change in electrical properties is dependent upon the nucleotide sequence, and can be measured. The nanopore can comprise a protein molecule, or it can be solid-state. One advantage of nanopore-based methods is that very long read lengths can be achieved, e.g. thousands, tens of thousands or hundreds of thousands of consecutive nucleotides can be read from a single molecule. Methods of polynucleotide characterization via nanopores are discussed for example, in U.S. Patent Application Publication Nos. 2006/0063171, U.S. 2006/0068401, and U.S. 2005/0202444.

One method of sequencing is an SBS method referred to as pyrophosphate-based sequencing. In pyrophosphate-based sequencing sample DNA sequence and the extension primer subjected to a polymerase reaction in the presence of a nucleotide triphosphate whereby the nucleotide triphosphate will only become incorporated and release pyrophosphate (PPi) if it is complementary to the base in the target position, the nucleotide triphosphate being added either to separate aliquots of sample-primer mixture or successively to the same sample-primer mixture. The release of PPi is then detected to indicate which nucleotide is incorporated.

In an embodiment, a region of the sequence product is determined by annealing a sequencing primer to a region of the template nucleic acid, and then contacting the sequencing primer with a DNA polymerase and a known nucleotide triphosphate, i.e., dATP, dCTP, dGTP, dTTP, or an analog of one of these nucleotides. The sequence can be determined by detecting a sequence reaction byproduct, as is described below.

The sequence primer can be any length or base composition, as long as it is capable of specifically annealing to a region of the amplified nucleic acid template. No particular structure for the sequencing primer is required so long as it is able to specifically prime a region on the amplified template nucleic acid. Preferably, the sequencing primer is complementary to a region of the template that is between the sequence to be characterized and the sequence hybridizable to the anchor primer. The sequencing primer is extended with the DNA polymerase to form a sequence product. The extension is performed in the presence of one or more types of nucleotide triphosphates, and if desired, auxiliary binding proteins.

Incorporation of the dNTP is preferably determined by assaying for the presence of a sequencing byproduct. In a preferred embodiment, the nucleotide sequence of the sequencing product is determined by measuring inorganic pyrophosphate (PPi) liberated from a nucleotide triphosphate (dNTP) as the dNMP is incorporated into an extended sequence primer. This method of sequencing, termed Pyrosequencing™ technology (PyroSequencing AB, Stockholm, Sweden) can be performed in solution (liquid phase) or as a solid phase technique. PPi-based sequencing methods are described generally in, e.g., WO9813523A1, Ronaghi, et al., 1996. Anal. Biochem. 242: 84-89, Ronaghi, et al., 1998. Science 281: 363-365 (1998) and U.S. Patent Application Publication No. 2001/0024790. See also , e.g., U.S. Patent Nos. 6,210,891 and 6,258,568.

In a preferred embodiment, DNA sequencing is performed using 454 corporation's (454 Life Sciences) sequencing apparatus and methods which are disclosed in copending patent applications USSN: 10/768,729, USSN: 10/767,779, USSN: 10/767,899, and USSN: 10/767,894 - all of which are filed January 28, 2004.

Unless otherwise defined, all technical and scientific terms used herein have the same meaning as commonly understood by one of ordinary skill in the art to which this invention belongs. Commonly understood definition would include those defined in USSN: 60/476,602, filed June 6, 2003; USSN: 60/476,504, filed June 6, 2003; USSN: 60/443,471, filed January 29, 2003; USSN: 60/476,313, filed June 6, 2003; USSN: 60/476,592, filed June 6, 2003; USSN: 60/465,071, filed April 23, 2003; USSN: 60/497,985, filed August 25, 2003; USSN: 10/767,779 filed January 28, 2004; 10/767,899 filed January 28, 2004; USSN: 10/767,894 filed January 28, 2004.

## EXAMPLES

## Example 1 Sequencing of the HLA locus

Five PCR primer pairs were designed to span known, publicly disclosed SNPs in the MHC class II locus. Primers were design using the Primer3 software (Whitehead Institute for Biomedical Research) using approx. 200 base-pair long genomic sequences encompassing the target regions as input. Each primer consisted of a locus specific 3' portion ranging in length from 20 to 24 bases and a constant 19 base 5' portion (shown in lowercase) that includes a 4 base key (high-lighted in bold). Primers were purchased from Integrated DNA Technologies (Coralville, IA):

SAD1F-DC1 gcctccctcgcgcca **tcag** ACCTCCCTCTGTGTCCTTACAA (SEQ ID NO:1)
SAD1R-DC1 gccttgccagcccgc **tcag** GGAGGGAATCATACTAGCACCA (SEQ ID NO:2)

SAD1F-DD14 gcctccctcgcgcca **tcag** TCTGACGATCTCTGTCTTCTAACC (SEQ ID NO:3)
SAD1R-DD14 gccttgccagcccgc **tcag** GCCTTGAACTACACGTGGCT (SEQ ID NO:4)

SAD1F-DE15 gcctccctcgcgcca **tcag** ATTTCTCTACCACCCCTGGC (SEQ ID NO:5)
SAD1R-DE15 gccttgccagcccgc **tcag** AGCTCATGTCTCCCGAAGAA (SEQ ID NO:6)

SAD1F-GA9 gcctccctcgcgcca **tcag** AAAGCCAGAAGAGGAAAGGC (SEQ ID NO:7)
SAD1R-GA9 gccttgccagcccgc **tcag** CTTGCAGATTGGTCATAAGG (SEQ ID NO:8)

SAD1F-F5 gcctccctcgcgcca **tcag** ACAGTGCAAACACCACCAAA (SEQ ID NO:9)
SAD1R-F5 gccttgccagcccgc **tcag** CCAGTATTCATGGCAGGGTT (SEQ ID NO:10)

Human genomic DNA (Cornell Medical Institute for Research, Camden, NJ) from 4 individuals was quantitated based on optical density at 260 nm and 100 ng (approx. 15,000 haploid genome equivalents) was used as template for each PCR amplification reaction. PCR reactions were performed under standard reaction conditions (60 mM Tris-SO$_4$, pH 8.9, 18 mM (NH$_4$)$_2$SO$_4$, 2.5 mM MgSO$_4$, 1 mM dNTPs, 0.625 uM of each primer, 4.5 units Platinum Taq High Fidelity polymerase (Invitrogen, Carlsbad, CA)) with the following temperature profile: 3 min 94 °C; 30 cycles of 30 s 94 °C, 45 s 57 °C, 1 min 72 °C; 3 min 72 °C. Amplification products were purified using a QiaQuick PCR Purification kit (Qiagen, Valencia, CA), and their anticipated sizes (156 to 181 base pairs) were verified on a 2100 BioAnalyzer microfluidics instrument using a 500 DNA LabChip® (Agilent Technologies, Inc, Palo Alto, CA). The purified amplicons were quantitated with a PicoGreen® dsDNA quantitation kit (Molecular Probes, Eugene, OR) and diluted to $10^7$ copies per microliter.
EBCA (Emulsion Based Clonal Amplification) was performed as described above with 0.5 amplicons per bead, using amplification primers SAD1F (GCC TCC CTC GCG CCA (SEQ ID NO:11)) and SAD1R and Sepharose capture beads with SADR1 (GCC TTG CCA GCC CGC (SEQ ID NO:12)) capture primer (Amersham BioSciences, Piscataway, NJ). All further manipulations, including breaking of the emulsions and sequencing on the PicoTiter plate were performed as described above.

**Example 2 Sensitive Mutation Detection**

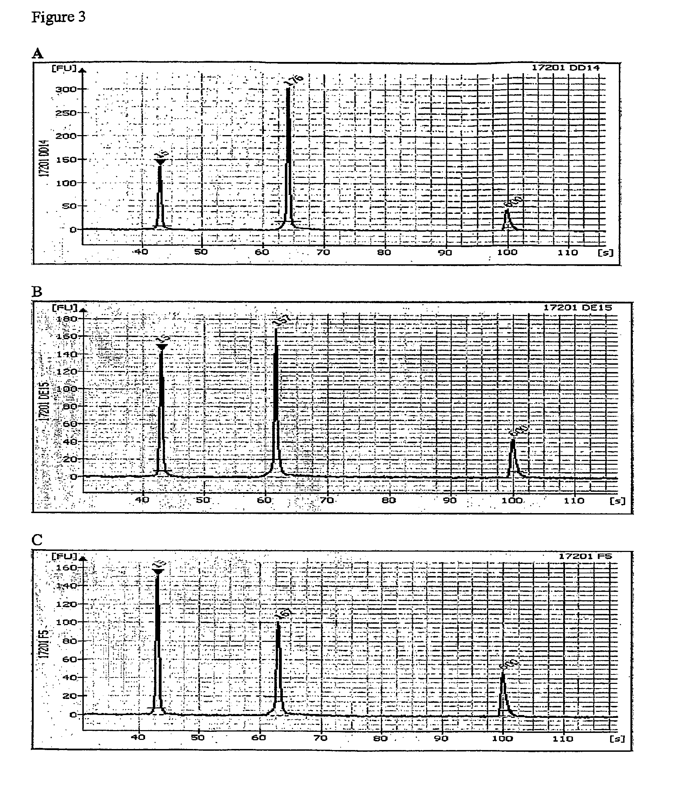

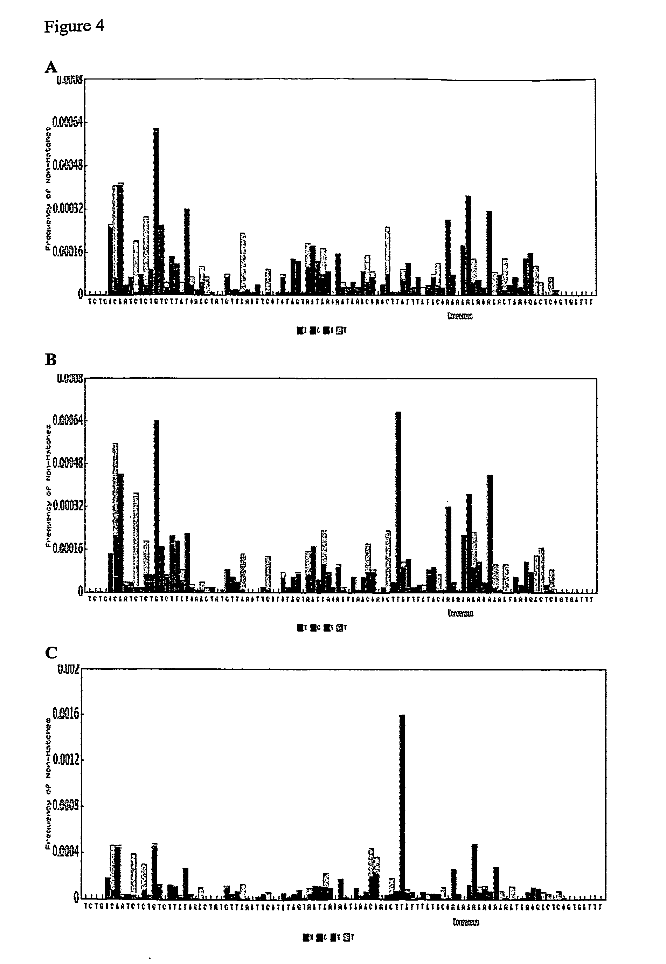

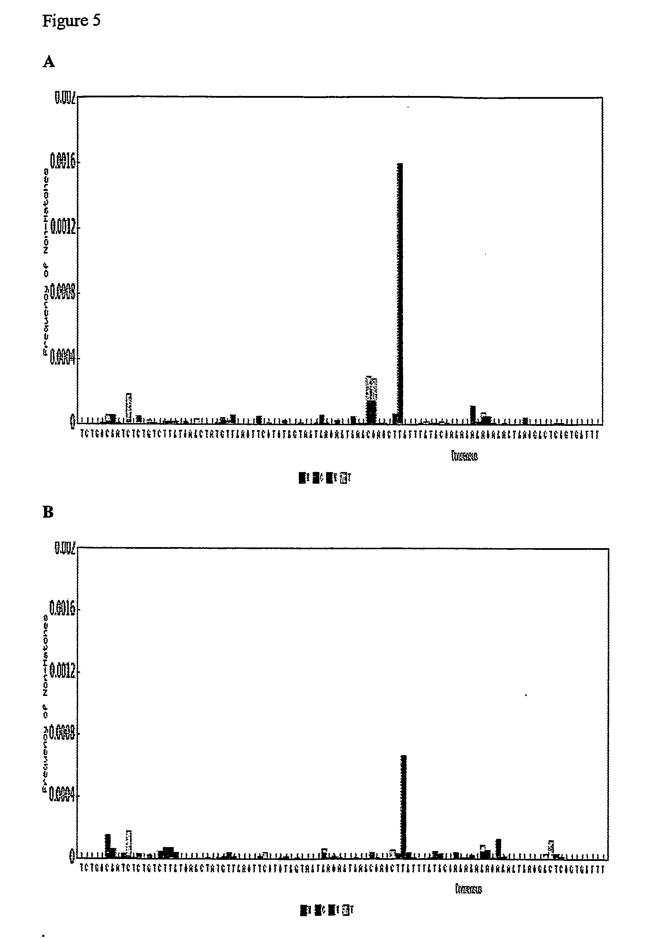

To demonstrate the capability of the current system (i.e., the 454 platform) to detect low abundance sequence variants, specifically single base substitutions, experiments were designed to sequence known alleles mixed at various ratios.
The 6 primer pairs listed above were tested for amplification efficiency and further analysis was performed using pairs SAD1F/R-DD14, SAD1F/R-DE15 and SAD1F/R-F5 which all produced distinct amplification products (Figure 3). A total of 8 human genomic DNA samples were amplified and sequenced on the 454 platform to determine the genotypes for each locus. To simplify the experimental setup all further analysis was done using primer pair SAD1F/R-DD14 (Figure 3A) and two samples shown to be homozygous for either the C or T allele at the particular locus.
The primary amplicons from each sample were quantitated and mixed at specific ratios ranging from 10:90 down to 1:1000, typically with the T allele in excess. After mixing the samples were diluted to a working concentration of 2 x $10^6$ copies per microliter and subjected to EBCA and sequenced on the 454 platform. Figure 2 presents sequencing data obtained from the mixing of the C allele in approximate ratios 1:500 and 1:1000 into the T allele. In both cases roughly 10,000 high-quality sequencing reads were generated and subjected to Blast analysis to identify nucleotide substitutions against a reference sequence (in this case the T allele carrying sequence). For visualization of the results the substitution frequency is plotted in a color-coded fashion relative to the reference sequence. The data demonstrate that in both samples the low frequency single base substitutions were readily identified (Figure 4A-C). Furthermore the background was found to be relatively consistent between samples allowing background subtraction. This typically produced a signal-to-noise ratio even for the 1:1000 allele that exceeded 10 (Figure 5A and B). Additional experimentation using samples of known genotypes has confirmed the ability to detect single nucleotide substitutions down to at least a 0.1 % abundance level. Additional confidence in low abundance changes can be obtained from sequencing a template in both directions. Typically the difference between the frequencies from the two independent bidirectional data sets is within 20% down to the 1% abundance level.

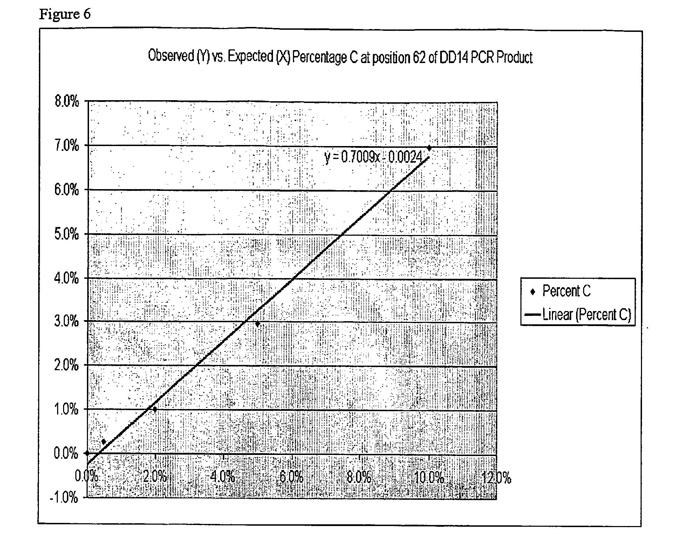

To demonstrate a linear response over a broader range of allelic ratios, amplicons representing the T and C alleles from the DD14 HLA locus were mixed in ratios 1:10, 1:20, 1:50 and 1:200 (10%, 5%, 2% and 0.5%), EBCA amplified and sequenced. Figure 6 shows that a linear increase in the relative number of low frequency allele was observed throughout the range ($R^2$=0.9927). The recorded absolute frequencies somewhat deviated from the intended ratios (See Table below) and were attributed to commonly observed difficulties trying to precisely quantitate, aliquot and mix small amounts of DNA.

| Expected Percent C | Total Reads | Expected C | Observed C | Observed T | Observed Percent C |
|---|---|---|---|---|---|
| 0.00% | 101450 | 0 | 1 | 101449 | 0.00% |
| 0.50% | 72406 | 361 | 193 | 72213 | 0.27% |
| 2.00% | 103292 | 2045 | 1049 | 102243 | 1.02% |
| 2.00% | 57115 | 1131 | 578 | 56537 | 1.01% |
| 5.00% | 112378 | 5452 | 3340 | 109038 | 2.97% |
| 10.00% | 104906 | 9760 | 7311 | 97595 | 6.97% |
| Summary of sequencing used to generate plot in Figure 6. Numbers in columns 2-5 indicate total number of sequenced templates, and the expected and observed numbers for each allele respectively. | | | | | |

**Example 3 Bacterial 16S Project - A Method to Examine Bacteria Populations**

Bacterial population surveys are essential applications for many fields including industrial process control, in addition to medical, environmental and agricultural research. One common method utilizes the 16S ribosomal RNA gene sequence to distinguish bacterial species (Jonasson, Olofsson et al. 2002; Grahn, Olofsson et al. 2003). Another method similarly examines the intervening sequence between the 16S and 23S ribosomal RNA genes (Garcia-Martinez, Bescos et al. 2001). However, the majority of researchers find a complete census of complex bacterial populations is impossible using current sample preparation and sequencing technologies; the labor requirements for such a project are either prohibitively expensive or force dramatic subsampling of the populations.

Currently, high throughput methods are not routinely used to examine bacterial populations. Common practice utilizes universal primer(s) to amplify the 16S ribosomal RNA gene (or regions within the gene), which are subsequently subcloned into vectors and sequenced. Restriction digests are often conducted on the vectors in an effort to reduce the sequencing load by eliminating vectors which exhibit identical restriction patterns. Resultant sequences are compared to a database of known genes from various organisms; estimates of population composition are drawn from the presence of species- or genus-specific gene sequences. The methods of this disclosure has the potential to revolutionize the study of bacterial populations by drastically reducing the labor costs through eliminating cloning and restriction digest steps, increasing informational output by providing complete sequences from the 16S (and possibly intergenic and 23S) RNA regions possibly allowing previously unobtainable substrain differentiation, and potentially providing estimates of species density by converting sequence oversampling into relative abundance.

One preferred method of nucleic acid sequencing is the pyrophosphate based sequencing methods developed by 454 Life Sciences. Utilization of the methods of the invention coupled with all aspects of the massively parallel 454 technology (some of which is disclosed in this specification) can greatly increase the throughput and reduce the cost of community identification. The 454 technology eliminates the need to clone large numbers of individual PCR products while the small size of the 16S gene (1.4kb) allows tens of thousands of samples to be processed simultaneously. The process has been successfully demonstrated in the manner outlined below.

Initially, *Escherichia coli* 16S DNA was obtained from *E. coli* TOP10 competent cells (Invitrogen, Carlsbad, CA.) transformed with the PCR2.1 vector, plated onto LB/Ampicillin plates (50 μg/ml) and incubated overnight at 37°C. A single colony was picked and inoculated into 3 ml of LB/Ampicillin broth and shaken at 250 RPM for 6 hours at 37°C. One microliter of this solution was used as template for amplifying the V1 and V3 regions of the 16S sequence.

Bipartite PCR primers were designed for two variable regions in the 16S gene, denoted V1 and V3 as described in Monstein et al (Monstein, Nikpour-Badr et al. 2001). Five prime tags comprised of 454 specific, 19 base (15 base amplification primers, followed by a 3', 4 base (TCGA) key) forward or reverse primers were fused to the region specific forward and reverse primers that flank the variable V1 and V3 regions. This may be represented as: 5' - (15 base forward or reverse Amplification primer) - (4 base key) - (forward or reverse V1 or V3 primer) - 3'. The primers used to produce 16S amplicons contain the following sequences, with the sequences in capital letter representing the V1 or V3 specific primers, the four bases in bold identify the key, and the lower case bases indicate the 454 amplification primers:

SAD-V1 fusion (forward): gcctccctcgcgcca **tcag** GAAGAGTTTGATCATGGCTCAG (SEQ ID NO:13)
SAD-V1 fusion (reverse): gccttgccagcccgc **tcag** TTACTCACCCGTCCGCCACT (SEQ ID NO:14)
SAD-V3 fusion (forward): gcctccctcgcgcca **tcag** GCAACGCGAAGAACCTTACC (SEQ ID NO:15)
SAD-V3 fusion (reverse): gccttgccagcccgc **tcag** ACGACAGCCATGCAGCACCT (SEQ ID NO:16)

The V1 and V3 amplicons were generated separately in PCR reactions that contained the following reagents: 1X HiFi buffer, 2.5 mM $MgSO_4$ (Invitrogen), 1 mM dNTPs (Pierce, Milwaukee WI.), 1$\mu$M each forward and reverse bipartite primer for either V1 or V3 regions (IDT, Coralville, IA), 0.15 U/$\mu$l Platinum HiFi Taq (Invitrogen). One microliter of *E. coli* LB/Ampicillin broth was added to the reaction mixture and 35 cycles of PCR were performed (94°C for 30 seconds, 55°C for 30 seconds, and 68°C for 150 seconds, with the final cycle followed by a 10°C infinite hold). Subsequently, 1 $\mu$l of the amplified reaction mix was run on the Agilent 2100 Bioanalyzer (Agilent, Palo Alto, CA) to estimate the concentration of the final product, and assure the proper size product 155 bp for the V1, 145 bp for the V3) was generated.

The V1 and V3 products were then combined, emulsified at template concentrations ranging from 0.5 to 10 template molecules per DNA capture bead and amplified through the EBCA (Emulsion Based Clonal Amplification) process as outlined in the EBCA Protocol section below. The resulting clonally amplified beads were subsequently sequenced on the 454 Genome Sequencer (454 Life Sciences, Branford CT).

The sequences obtained from the amplified beads were aligned against the *Escherichia coli* 16S gene sequence (Entrez gi174375). Acceptable (or "mapped") alignments were distinguished from rejected (or "unmapped") alignments by calculating the alignment score for each sequence. The score is the average logarithm of the probability that an observed signal corresponds to the expected homopolymer, or:

$$S = \sum \ln[P(s|h)]/N$$

where S is the computed alignment score, P is the probability at a specific flow, s is the signal measured at that flow, h is the length of the reference homopolymer expected at that flow, and N is the total number of flows aligned. The alignment score for each sequence was then compared to a Maximum Alignment Score, or MAS; alignments scoring less than the MAS were considered "real" and were printed to the output file. For this project, a MAS of 1.0 (roughly equivalent to 95% identity) was used.

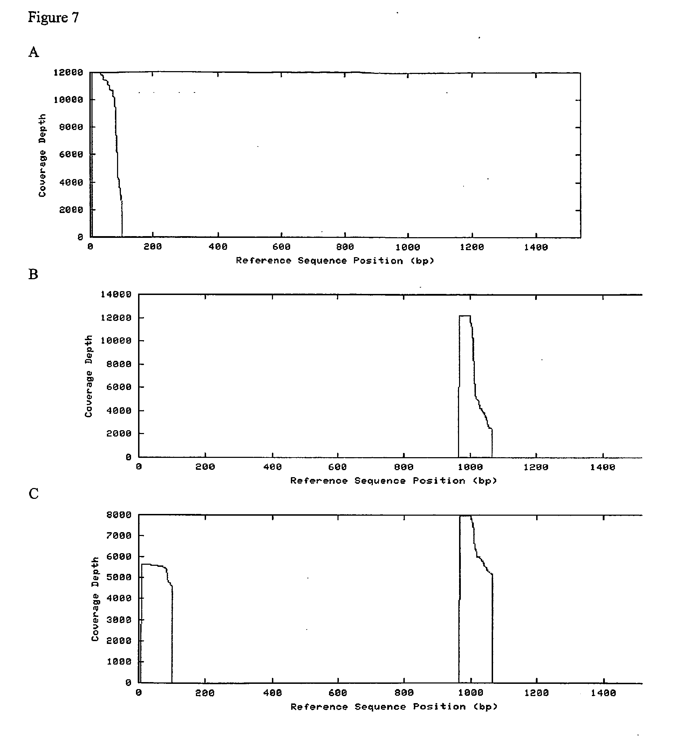

For the sequences generated with the V1 specific primers, of the 13702 sequences generated, 87.75% or 11973 reads mapped to the genome with an alignment score less than 1.0, and a read length greater than 21 bases. A graphical display showing the location of the reads mapping to the 1.6 Kb 16S gene fragment is shown in Figure 7A, indicating roughly 12,000 reads mapping to the first 100 bases of the 16S gene.

BLASTing the unmodified consensus sequence (AAGAGTTTtGATCATGGCTCAGATTGAACGCTGGCGGCAGGCCTAACACATGCA AGTCGA ACGGTAACAGGA (SEQ ID NO:17)) against the 16S database (http://greengenes.llnl.gov) matched *Escherichia coli* as the first known organism

```
>lcl|009704 X80724 Escherichia coli str. Seattle 1946 ATCC 25922.
        Length = 1452
 Score =  125 bits (63), Expect = 1e-28
 Identities = 70/71 (98%), Gaps = 1/71 (1%)
 Strand = Plus / Plus
Query: 7   tttgatcatggctcagattgaacgctggcggcaggcctaacacatgcaagtcgaacggta 66
           ||||||||||||||||||||||||||||||||||||||||||||||||||||||||||||||
Sbjct: 3   tttgatcatggctcagattgaacgctggcggcaggcctaacacatgcaagtcgaacggta 62

Query: 67  acgaggaacga 77 (SEQ ID NO:18)
           ||  |||||||||
Sbjct: 63  ac-aggaacga 72 (SEQ ID NO:19)

>lcl|090202 AY319393 Escherichia coli strain 5.2 16S ribosomal RNA
        gene, partial sequence
        Length = 1399
 Score =  123 bits (62), Expect = 5e-28
 Identities = 62/62 (100%)
 Strand = Plus / Plus
```

The V1 consensus sequence was edited to AAGAGTTT<u>T</u>GATCATGGCTCAGATTGAACGCTGGCGGCAG-

GCCTAACACATGCA AGTCGAACGGTAACAGGA (SEQ ID NO:20), as the fourth "T" at position 9 (marked in bold and underline) of a homopolymer stretch was reviewed and removed, based on an exceedingly low confidence score. The BLAST results of the edited V1 sequence demonstrated improved hits against *Escherichia coli* 16S genes.

```
>lcl|076948 AE016770 Escherichia coli CFT073 section 16 of 18 of the complete
genome
          Length = 1542
Score =  141 bits (71), Expect = 1e-33
 Identities = 71/71 (100%)
 Strand = Plus / Plus
Query: 1   aagagtttgatcatggctcagattgaacgctggcggcaggcctaacacatgcaagtcgaa 60
           ||||||||||||||||||||||||||||||||||||||||||||||||||||||||||||
Sbjct: 6   aagagtttgatcatggctcagattgaacgctggcggcaggcctaacacatgcaagtcgaa 65


Query: 61  cggtaacagga 71 (SEQ ID NO:21)
           |||||||||||
Sbjct: 66  cggtaacagga 76 (SEQ ID NO:22)
```

Similar results were obtained with the V3 specific primers. Of the 17329 reads, 71.00% mapped to the 16S reference genome under identical analysis conditions as used with the V1 templates above. This is a lower number than the 87.75% of V1 reads that mapped, and this may reveal a greater diverge between the V3 sample and reference sequences than between the V1 sample and reference sequences. The consensus sequence: CAACGCGAAGAACCTTACCTGGTCTTGACATCCACGAAGTTAC<u>T</u>AGAGATGAG AATGTGCCGTTCGGGAACCG-GTGAGACAGGTGCTGCATGGCTGTCGTCTg (SEQ ID NO:23), mapped to regions 966-1067 of the reference genome as shown in Figure 7B.

Unlike the V1 sequence BLAST results from the unmodified consensus sequence did not match *Escherichia coli* as the first known organism, but rather as the second organism.

```
>lcl|088104 AJ567617 Escherichia coli partial 16S rRNA gene, clone
          MBAE104
          Length = 1497
Score =  147 bits (74), Expect = 3e-35
 Identities = 98/102 (96%), Gaps = 3/102 (2%)
 Strand = Plus / Plus
Query: 1     caacgcgaagaaccttacctggtcttgacatccacgaagtttactagagatgagaatgtg 60
             ||||||||||||||||||||||||||||||||||||||||||||||| | |||||||||||||
Sbjct: 956   caacgcgaagaaccttacctggtcttgacatccacgaagttttc-agagatgagaatgtg 1014


Query: 61    ccgttcgggaaccggtgagacaggtgctgcatggctgtcgtc 102 (SEQ ID NO:24)
             || |||||||||| ||||||||||||||||||||||||||||
Sbjct: 1015  cc-ttcgggaacc-gtgagacaggtgctgcatggctgtcgtc 1054 (SEQ ID NO:25)
```

The consensus sequence was reviewed and edited to CAACGCGAAGAACCTTACCTGGTCTTGACATCCACGAAGTTTACAGAGATGAGA ATGTGCCGTTCGGGAACCGTGAGACAGGTGCTGCATGGCTGTCGTCTg (SEQ ID NO:26)(with the removal of two bases) based on the confidence scores, and reBLASTed. The BLAST resulted in the highest ranked hit occurring against *E. coli*.

```
>lcl|088104 AJ567617 Escherichia coli partial 16S rRNA gene, clone
          MBAE104
          Length = 1497
 Score =  174 bits (88), Expect = 1e-43
 Identities = 98/100 (98%), Gaps = 1/100 (1%)
 Strand = Plus / Plus
Query: 1     caacgcgaagaaccttacctggtcttgacatccacgaagtttacagagatgagaatgtgc 60
             ||||||||||||||||||||||||||||||||||||||||||||| ||||||||||||||||
Sbjct: 956   caacgcgaagaaccttacctggtcttgacatccacgaagttttcagagatgagaatgtgc 1015


Query: 61    cgttcgggaaccgtgagacaggtgctgcatggctgtcgtc 100 (SEQ ID NO:27)
             | |||||||||||||||||||||||||||||||||||||||
Sbjct: 1016  c-ttcgggaaccgtgagacaggtgctgcatggctgtcgtc 1054 (SEQ ID NO:28)
```

A second experiment was conducted to demonstrate the ability to use mixed PCR primers on unprocessed bacterial cells, where the *E. coli* cells were grown to saturation and 1 μl of a 1:1000 dilution of the bacterial broth was added to the EBCA reaction mix in lieu of template. The primers used in the EBCA reaction consisted of V1- and V3-specific bipartite primers at 0.04 μM each, as well as the forward and reverse 454 amplification primers at 0.625 and 0.04 μM respectively. Otherwise, the EBCA protocol outlined below was followed.

The data showed that V1 and V3 regions could be successfully amplified, sequenced and distinguished simultaneously from an untreated pool of bacterial cells. Of the 15484 reads, 87.66% mapped to the 16S reference genome, with the sequences located at the distinctive V1 and V3 positions shown in Figure 7C.

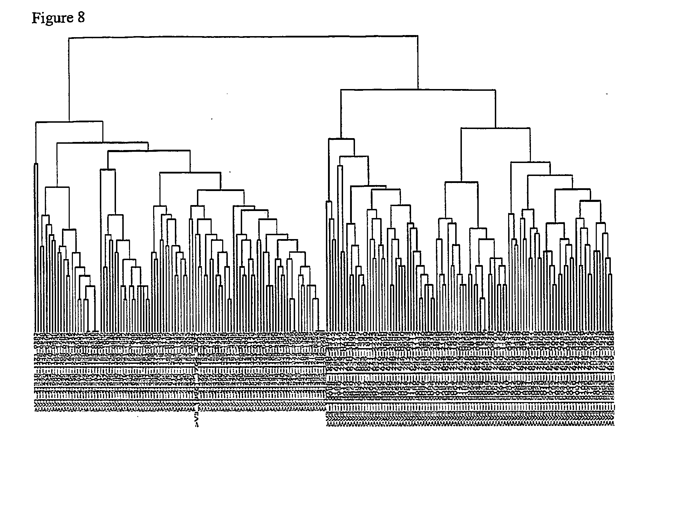

The ability to distinguish between V1 and V3 sequences was assessed by pooling 100 reads of both V1 and V3 sequences, and converting the raw signal data into a binary string, with a "1" indicating that a base was present at a given flow, and a "0" indicating that it was absent. Homopolymer stretches were collapsed into a single positive value, so that "A", "AA", and "AAAAA" (SEQ ID NO:29) all received an identical score of "1 ". The collapsed binary strings were then clustered via the Hierarchical Ordered Partitioning and Collapsing Hybrid (HOPACH) methodology (Pollard and van der Laan 2005) in the R statistical package (Team 2004). The resulting phylogenetic tree, shown in Figure 8, clearly discriminates between the V1 (shorter length red labels) and the V3 (longer length blue labels) sequences in all but 1 of the 200 sequences.

The ability to discriminate this clearly between two similar regions from the same gene within the same organism suggest that this technology should prove adept at discriminating between variable regions from distinct organisms, providing a valuable diagnostic tool.

**Example 4 EBCA Protocol**

*4.1 Preparation of DNA Capture Beads*

Packed beads from a 1 mL N-hydroxysuccinimide ester (NHS)-activated Sepharose HP affinity column (Amersham Biosciences, Piscataway, NJ) were removed from the column and activated as described in the product literature (Amersham Pharmacia Protocol # 71700600AP). Twenty-five microliters of a 1 mM amine-labeled HEG capture primer (5'-Amine-3 sequential 18-atom hexa-ethyleneglycol spacers CCATCTGTTGCGTGCGTGTC-3' (SEQ ID NO:30)) (IDT Technologies, Coralville, IA, USA) in 20 mM phosphate buffer, pH 8.0, were bound to the beads, after which 25-36 μm beads were selected by serial passage through 36 and 25 μm pore filter mesh sections (Sefar America, Depew, NY, USA). DNA capture beads that passed through the first filter, but were retained by the second were collected in bead storage buffer (50 mM Tris, 0.02% Tween, 0.02% sodium azide, pH 8), quantitated with a Multisizer 3 Coulter Counter (Beckman Coulter, Fullerton, CA, USA) and stored at 4°C until needed.

*4.2 Binding Template Species to DNA Capture Beads*

Template molecules were annealed to complementary primers on the DNA Capture beads in a UV-treated laminar flow hood. Six hundred thousand DNA capture beads suspended in bead storage buffer were transferred to a 200 μL PCR tube, centrifuged in a benchtop mini centrifuge for 10 seconds, the tube rotated 180° and spun for an additional 10 seconds to ensure even pellet formation. The supernatant was then removed, and the beads washed with 200 μL of Annealing Buffer (20 mM Tris, pH 7.5 and 5 mM magnesium acetate), vortexed for 5 seconds to resuspend the beads, and pelleted as above. All but approximately 10 μL of the supernatant above the beads were removed, and an additional 200 μL of Annealing Buffer were added. The beads were vortexed again for 5 seconds, allowed to sit for 1 minute, then pelleted as above. All but 10 μL of supernatant were discarded, and 0.48 μL of 2 x 10$^7$ molecules per μL template library were added to the beads. The tube was vortexed for 5 seconds to mix the contents, after which the templates were annealed to the beads in a controlled denaturation/annealing program preformed in an MJ thermocycler (5 minutes at 80 °C, followed by a decrease by 0.1 °C /sec to 70 °C, 1 minute at 70 °C, decrease by 0.1 °C /sec to 60 °C, hold at 60 °C for 1 minute, decrease by 0.1 °C /sec to 50 °C, hold at 50 °C for 1 minute, decrease by 0.1 °C /sec to 20 °C, hold at 20 °C). Upon completion of the annealing process the beads were stored on ice until needed.

*4.3 PCR Reaction Mix Preparation and Formulation*

To reduce the possibility of contamination, the PCR reaction mix was prepared in a in a UV-treated laminar flow hood located in a PCR clean room. For each 600,000 bead emulsion PCR reaction, 225 μL of reaction mix (1X Platinum HiFi Buffer (Invitrogen), 1mM dNTPs (Pierce), 2.5 mM MgSO$_4$ (Invitrogen), 0.1% Acetylated, molecular biology grade BSA (Sigma), 0.01% Tween-80 (Acros Organics), 0.003 U/μL thermostable pyrophosphatase (NEB), 0.625 μM forward (5' - CGTTTCCCCTGTGTGCCTTG-3' (SEQ ID NO:31)) and 0.039 μM reverse primers (5'-CCATCTGTTGCG TGCGTGTC-3' (SEQ ID NO:32)) (IDT Technologies, Coralville, IA, USA) and 0.15 U/μL Platinum Hi-Fi Taq Polymerase (Invitrogen)) were prepared in a 1.5 mL tube. Twenty-five microliters of the reaction mix were removed and stored in an individual 200 μL PCR tube for use as a negative control. Both the reaction mix and negative controls were stored on ice until needed. Additionally, 240 μL of mock amplification mix (1X Platinum HiFi Buffer (Invitrogen), 2.5 mM MgSO$_4$ (Invitrogen), 0.1 % BSA, 0.01% Tween) for every emulsion were prepared in a 1.5 mL tube, and similarly stored at room temperature until needed.

*4.4 Emulsification and Amplification*

The emulsification process creates a heat-stable water-in-oil emulsion with approximately 10,000 discrete PCR microreactors per microliter which serve as a matrix for single molecule, clonal amplification of the individual molecules of the target library. The reaction mixture and DNA capture beads for a single reaction were emulsified in the following manner: in a UV-treated laminar flow hood, 200 μL of PCR solution were added to the tube containing the 600,000 DNA capture beads. The beads were resuspended through repeated pipette action, after which the PCR-bead mixture was permitted to sit at room temperature for at least 2 minutes, allowing the beads to equilibrate with the PCR solution. Meanwhile, 400 μL of Emulsion Oil (60 % (w/w) DC 5225C Formulation Aid (Dow Chemical CO, Midland, MI), 30% (w/w) DC 749 Fluid (Dow Chemical CO, Midland, MI), and 30% (w/w) Ar20 Silicone Oil (Sigma)) were aliquotted into a flat-topped 2 mL centrifuge tube (Dot Scientific). The 240 μL of mock amplification mix were then added to 400 μL of emulsion oil, the tube capped securely and placed in a 24 well TissueLyser Adaptor (Qiagen) of a TissueLyser MM300 (Retsch GmbH & Co. KG, Haan, Germany). The emulsion was homogenized for 5 minutes at 25 oscillations/sec to generate the extremely small emulsions, or "microfines", that confer additional stability to the reaction.

During the microfine formation, 160 μL of the PCR amplification mix were added to the mixture of annealed templates and DNA capture beads. The combined beads and PCR reaction mix were briefly vortexed and allowed to equilibrate for 2 minutes. After the microfines had been formed, the amplification mix, templates and DNA capture beads were added to the emulsified material. The TissueLyser speed was reduced to 15 oscillations per second and the reaction mix homogenized for 5 minutes. The lower homogenization speed created water droplets in the oil mix with an average diameter of 100 to 150 μm, sufficiently large to contain DNA capture beads and amplification mix.

The emulsion was aliquotted into 7 to 8 separate PCR tubes each containing roughly 80 μL. The tubes were sealed and placed in a MJ thermocycler along with the 25 μl negative control made previously. The following cycle times were used: 1X (4 minutes at 94°C) - Hotstart Initiation, 40X (30 seconds at 94°C, 60 seconds at 58°C, 90 seconds at 68°C) - Amplification, 13X (30 seconds at 94 °C, 360 seconds at 58 °C) - Hybridization Extension. After completion of the PCR program, the reactions were removed and the emulsions either broken immediately (as described below) or the reactions stored at 10°C for up to 16 hours prior to initiating the breaking process.

*4.5 Breaking the Emulsion and Recovery of Beads*

Fifty microliters of isopropyl alcohol (Fisher) were added to each PCR tube containing the emulsion of amplified material, and vortexed for 10 seconds to lower the viscosity of the emulsion. The tubes were centrifuged for several seconds in a microcentrifuge to remove any emulsified material trapped in the tube cap. The emulsion-isopropyl alcohol mix was withdrawn from each tube into a 10 mL BD-Disposable Syringe (Fisher Scientific) fitted with a blunt 16 gauge blunt needle (Brico Medical Supplies). An additional 50 μL of isopropyl alcohol were added to each PCR tube, vortexed, centrifuged as before, and added to the contents of the syringe. The volume inside the syringe was increased to 9 mL with isopropyl alcohol, after which the syringe was inverted and 1 mL of air was drawn into the syringe to facilitate mixing the isopropanol and emulsion. The blunt needle was removed, a 25 mm Swinlock filter holder (Whatman) containing 15 μm pore Nitex Sieving Fabric (Sefar America, Depew, NY, USA) attached to the syringe luer, and the blunt needle affixed to the opposite side of the Swinlock unit.

The contents of the syringe were gently but completely expelled through the Swinlock filter unit and needle into a waste container with bleach. Six milliliters of fresh isopropyl alcohol were drawn back into the syringe through the blunt needle and Swinlock filter unit, and the syringe inverted 10 times to mix the isopropyl alcohol, beads and remaining emulsion components. The contents of the syringe were again expelled into a waste container, and the wash process repeated twice with 6 mL of additional isopropyl alcohol in each wash. The wash step was repeated with 6 mL of 80% Ethanol / 1X Annealing Buffer (80% Ethanol, 20 mM Tris-HCl, pH 7.6, 5 mM Magnesium Acetate). The beads were then washed with 6 mL of 1X Annealing Buffer with 0.1 % Tween (0.1 % Tween-20, 20 mM Tris-HCl, pH 7.6, 5 mM Magnesium Acetate), followed by a 6 mL wash with picopure water.

After expelling the final wash into the waste container, 1.5 mL of 1 mM EDTA were drawn into the syringe, and the Swinlock filter unit removed and set aside. The contents of the syringe were serially transferred into a 1.5 mL centrifuge tube. The tube was periodically centrifuged for 20 seconds in a minifuge to pellet the beads and the supernatant removed, after which the remaining contents of the syringe were added to the centrifuge tube. The Swinlock unit was reattached to the filter and 1.5 mL of EDTA drawn into the syringe. The Swinlock filter was removed for the final time, and the beads and EDTA added to the centrifuge tube, pelletting the beads and removing the supernatant as necessary.

*4.6 Second Strand Removal*

Amplified DNA, immobilized on the capture beads, was rendered single stranded by removal of the secondary strand through incubation in a basic melt solution. One mL of freshly prepared Melting Solution (0.125 M NaOH, 0.2 M NaCl) was added to the beads, the pellet resuspended by vortexing at a medium setting for 2 seconds, and the tube placed in a Thermolyne LabQuake tube roller for 3 minutes. The beads were then pelleted as above, and the supernatant carefully removed and discarded. The residual melt solution was then diluted by the addition of 1 mL Annealing Buffer (20 mM Tris-Acetate, pH 7.6, 5 mM Magnesium Acetate), after which the beads were vortexed at medium speed for 2 seconds, and the beads pelleted, and supernatant removed as before. The Annealing Buffer wash was repeated, except that only 800 μL of the Annealing Buffer were removed after centrifugation. The beads and remaining Annealing Buffer were transferred to a 0.2 mL PCR tube, and either used immediately or stored at 4°C for up to 48 hours before continuing with the subsequent enrichment process.

*4. 7 Enrichment of Beads*

Up to this point the bead mass was comprised of both beads with amplified, immobilized DNA strands, and null beads with no amplified product. The enrichment process was utilized to selectively capture beads with sequenceable amounts of template DNA while rejecting the null beads.

The single stranded beads from the previous step were pelleted by 10 second centrifugation in a benchtop mini centrifuge, after which the tube was rotated 180° and spun for an additional 10 seconds to ensure even pellet formation. As much supernatant as possible was then removed without disturbing the beads. Fifteen microliters of Annealing Buffer were added to the beads, followed by 2 μL of 100 μM biotinylated, 40 base HEG enrichment primer (5' Biotin - 18-atom hexa-ethyleneglycol spacer - CGTTTCCCCTGTGTGCCTTGCCATCTGTTCCCTCCCTGTC-3' (SEQ ID NO:33), IDT Technologies, complementary to the combined amplification and sequencing sites (each 20 bases in length) on the 3'-end of the bead-immobilized template. The solution was mixed by vortexing at a medium setting for 2 seconds, and the enrichment primers annealed to the immobilized DNA strands using a controlled denaturation/annealing program in an MJ thermocycler (30 seconds at 65°C, decrease by 0.1 °C /sec to 58°C, 90 seconds at 58°C, and a 10°C hold).

While the primers were annealing, a stock solution of SeraMag-30 magnetic streptavidin beads (Seradyn, Indianapolis, IN, USA) was resuspended by gentle swirling, and 20 μL of SeraMag beads were added to a 1.5 mL microcentrifuge tube containing 1 mL of Enhancing Fluid (2 M NaCl, 10 mM Tris-HCl, 1 mM EDTA, pH 7.5). The SeraMag bead mix was vortexed for 5 seconds, and the tube placed in a Dynal MPC-S magnet, pelletting the paramagnetic beads against the side of the microcentrifuge tube. The supernatant was carefully removed and discarded without disturbing the SeraMag beads, the tube removed from the magnet, and 100μL of enhancing fluid were added. The tube was vortexed for 3 seconds to resuspend the beads, and the tube stored on ice until needed.

Upon completion of the annealing program, 100 μL of Annealing Buffer were added to the PCR tube containing the DNA Capture beads and enrichment primer, the tube vortexed for 5 seconds, and the contents transferred to a fresh 1.5 mL microcentrifuge tube. The PCR tube in which the enrichment primer was annealed to the capture beads was washed once with 200 μL of annealing buffer, and the wash solution added to the 1.5 mL tube. The beads were washed three times with 1 mL of annealing buffer, vortexed for 2 seconds, pelleted as before, and the supernatant carefully removed. After the third wash, the beads were washed twice with 1 mL of ice cold enhancing fluid, vortexed, pelleted, and the supernatant removed as before. The beads were then resuspended in 150 μL ice cold enhancing fluid and the bead solution added to the washed SeraMag beads.

The bead mixture was vortexed for 3 seconds and incubated at room temperature for 3 minutes on a LabQuake tube roller, while the streptavidin-coated SeraMag beads bound to the biotinylated enrichment primers annealed to immobilized templates on the DNA capture beads. The beads were then centrifuged at 2,000 RPM for 3 minutes, after which the beads were gently "flicked" until the beads were resuspended. The resuspended beads were then placed on ice for 5 minutes. Following the incubation on ice, cold Enhancing Fluid was added to the beads to a final volume of 1.5 mL. The tube inserted into a Dynal MPC-S magnet, and the beads were left undisturbed for 120 seconds to allow the beads to pellet against the magnet, after which the supernatant (containing excess SeraMag and null DNA capture beads) was carefully removed and discarded.

The tube was removed from the MPC-S magnet, 1 mL of cold enhancing fluid added to the beads, and the beads resuspended with gentle flicking. It was essential not to vortex the beads, as vortexing may break the link between the SeraMag and DNA capture beads. The beads were returned to the magnet, and the supernatant removed. This wash was repeated three additional times to ensure removal of all null capture beads. To remove the annealed enrichment primers and SeraMag beads from the DNA capture beads, the beads were resuspended in 1 mL of melting solution, vortexed for 5 seconds, and pelleted with the magnet. The supernatant, containing the enriched beads, was transferred to a separate 1.5 mL microcentrifuge tube, the beads pelleted and the supernatant discarded. The enriched beads were then resuspended in 1X Annealing Buffer with 0.1% Tween-20. The beads were pelleted on the MPC again, and the supernatant transferred to a fresh 1.5 mL tube, ensuring maximal removal of remaining SeraMag beads. The beads were centrifuged, after which the supernatant was removed, and the beads washed 3 times with 1 mL of 1X Annealing Buffer. After the third wash, 800 μL of the supernatant were removed, and the remaining beads and solution transferred to a 0.2 mL PCR tube.

The average yield for the enrichment process was 33% of the original beads added to the emulsion, or 198,000 enriched beads per emulsified reaction. As the 60 x 60mm PTP format required 900,000 enriched beads, five 600,000 bead emulsions were processed per 60 x 60 mm PTP sequenced.

*4.8 Sequencing Primer Annealing*

The enriched beads were centrifuged at 2,000 RPM for 3 minutes and the supernatant decanted, after which 15 μL of annealing buffer and 3 μL of sequencing primer (100 mM SAD1F (5'- GCC TCC CTC GCG CCA-3' (SEQ ID NO:34), IDT Technologies), were added. The tube was then vortexed for 5 seconds, and placed in an MJ thermocycler for the following 4 stage annealing program: 5 minutes at 65 °C, decrease by 0.1 °C /sec to 50 °C, 1 minute at 50 °C, decrease by 0.1 °C /sec to 40 °C, hold at 40 °C for 1 minute, decrease by 0.1 °C /sec to 15 °C, hold at 15 °C.

Upon completion of the annealing program, the beads were removed from thermocycler and pelleted by centrifugation for 10 seconds, rotating the tube 180°, and spun for an additional 10 seconds. The supernatant was discarded, and 200 μL of annealing buffer were added. The beads were resuspended with a 5 second vortex, and the beads pelleted as before. The supernatant was removed, and the beads resuspended in 100 μL annealing buffer, at which point the beads were quantitated with a Multisizer 3 Coulter Counter. Beads were stored at 4 °C and were stable for at least one week.

*4.9 Incubation of DNA beads with Bst DNA polymerase, Large Fragment and SSB protein*

Bead wash buffer (100 ml) was prepared by the addition of apyrase (Biotage) (final activity 8.5 units/liter) to 1x assay buffer containing 0.1% BSA. The fiber optic slide was removed from picopure water and incubated in bead wash buffer. Nine hundred thousand of the previously prepared DNA beads were centrifuged and the supernatant was carefully removed. The beads were then incubated in 1290 μl of bead wash buffer containing 0.4 mg/mL polyvinyl pyrrolidone (MW 360,000), 1 mM DTT, 175 μg of *E. coli* single strand binding protein (SSB) (United States Biochemicals) and 7000 units of *Bst* DNA polymerase, Large Fragment (New England Biolabs). The beads were incubated at room temperature on a rotator for 30 minutes.

*4.10 Preparation of enzyme beads and micro particle fillers*

UltraGlow Luciferase (Promega) and *Bst* ATP sulfurylase were prepared in house as biotin carboxyl carrier protein (BCCP) fusions. The 87-aminoacid BCCP region contains a lysine residue to which a biotin is covalently linked during the *in vivo* expression of the fusion proteins in *E. coli.* The biotinylated luciferase (1.2 mg) and sulfurylase (0.4 mg) were premixed and bound at 4°C to 2.0 mL of Dynal M280 paramagnetic beads (10 mg/mL, Dynal SA, Norway) according to manufacturer's instructions. The enzyme bound beads were washed 3 times in 2000 μL of bead wash buffer and resuspended in 2000 μL of bead wash buffer.

Seradyn microparticles (Powerbind SA, 0.8 μm, 10 mg/mL, Seradyn Inc) were prepared as follows: 1050 μL of the stock were washed with 1000 μL of 1X assay buffer containing 0.1% BSA. The microparticles were centrifuged at 9300 g for 10 minutes and the supernatant removed. The wash was repeated 2 more times and the microparticles were resuspended in 1050 μL of 1X assay buffer containing 0.1% BSA. The beads and microparticles are stored on ice until use.

*4.11 Bead deposition*

The Dynal enzyme beads and Seradyn microparticles were vortexed for one minute and 1000 μL of each were mixed in a fresh microcentrifuge tube, vortexed briefly and stored on ice. The enzyme / Seradyn beads (1920 μl) were mixed with the DNA beads (1300 μl) and the final volume was adjusted to 3460 μL with bead wash buffer. Beads were deposited in ordered layers. The fiber optic slide was removed from the bead wash buffer and Layer 1, a mix of DNA and enzyme/Seradyn beads, was deposited. After centrifuging, Layer 1 supernatant was aspirated off the fiber optic slide and Layer 2, Dynal enzyme beads, was deposited. This section describes in detail how the different layers were centrifuged.

Layer 1. A gasket that creates two 30x60 mm active areas over the surface of a 60x60 mm fiber optic slide was carefully fitted to the assigned stainless steel dowels on the jig top. The fiber optic slide was placed in the jig with the smooth unetched side of the slide down and the jig top/gasket was fitted onto the etched side of the slide. The jig top was then properly secured with the screws provided, by tightening opposite ends such that they are finger tight. The DNA-enzyme bead mixture was loaded on the fiber optic slide through two inlet ports provided on the jig top. Extreme care was taken to minimize bubbles during loading of the bead mixture. Each deposition was completed with one gentle continuous thrust of the pipette plunger. The entire assembly was centrifuged at 2800 rpm in a Beckman Coulter Allegra 6 centrifuge with GH 3.8-A rotor for 10 minutes. After centrifugation the supernatant was removed with a pipette.

Layer 2. Dynal enzyme beads (920 $\mu$L) were mixed with 2760 $\mu$L of bead wash buffer and 3400 $\mu$L of enzyme-bead suspension was loaded on the fiber optic slide as described previously. The slide assembly was centrifuged at 2800 rpm for 10 min and the supernatant decanted. The fiber optic slide is removed from the jig and stored in bead wash buffer until it is ready to be loaded on the instrument.

*4.12 Sequencing on the 454 Instrument*

All flow reagents were prepared in 1x assay buffer with 0.4 mg/mL polyvinyl pyrrolidone (MW 360,000), 1 mM DTT and 0.1% Tween 20. Substrate (300 $\mu$M D-luciferin (Regis) and 2.5 $\mu$M adenosine phosphosulfate (Sigma)) was prepared in 1X assay buffer with 0.4 mg/mL polyvinyl pyrrolidone (MW 360,000), 1 mM DTT and 0.1 % Tween 20. Apyrase wash is prepared by the addition of apyrase to a final activity of 8.5 units per liter in 1X assay buffer with 0.4 mg/mL polyvinyl pyrrolidone (MW 360,000), 1 mM DTT and 0.1 % Tween 20. Deoxynucleotides dCTP, dGTP and dTTP (GE Biosciences) were prepared to a final concentration of 6.5 $\mu$M, $\alpha$-thio deoxyadenosine triphosphate (dATP$\alpha$S, Biolog) and sodium pyrophosphate (Sigma) were prepared to a final concentration of 50 $\mu$M and 0.1 $\mu$M, respectively, in the substrate buffer.

The 454 sequencing instrument consists of three major assemblies: a fluidics subsystem, a fiber optic slide cartridge/flow chamber, and an imaging subsystem. Reagents inlet lines, a multi-valve manifold, and a peristaltic pump form part of the fluidics subsystem. The individual reagents are connected to the appropriate reagent inlet lines, which allows for reagent delivery into the flow chamber, one reagent at a time, at a pre-programmed flow rate and duration. The fiber optic slide cartridge/flow chamber has a 250 $\mu$m space between the slide's etched side and the flow chamber ceiling. The flow chamber also included means for temperature control of the reagents and fiber optic slide, as well as a light-tight housing. The polished (unetched) side of the slide was placed directly in contact with the imaging system.

The cyclical delivery of sequencing reagents into the fiber optic slide wells and washing of the sequencing reaction byproducts from the wells was achieved by a pre-programmed operation of the fluidics system. The program was written in a form of an Interface Control Language (ICL) script, specifying the reagent name (Wash, dATP$\alpha$S, dCTP, dGTP, dTTP, and PPi standard), flow rate and duration of each script step. Flow rate was set at 4 mL/min for all reagents and the linear velocity within the flow chamber was approximately ~1 cm/s. The flow order of the sequencing reagents were organized into kernels where the first kernel consisted of a PPi flow (21 seconds), followed by 14 seconds of substrate flow, 28 seconds of apyrase wash and 21 seconds of substrate flow. The first PPi flow was followed by 21 cycles of dNTP flows (dC-substrate-apyrase wash-substrate dA-substrate-apyrase wash-substrate-dG-substrate-apyrase wash-substrate-dT-substrate-apyrase wash-substrate), where each dNTP flow was composed of 4 individual kernels. Each kernel is 84 seconds long (dNTP-21 seconds, substrate flow-14 seconds, apyrase wash-28 seconds, substrate flow-21 seconds); an image is captured after 21 seconds and after 63 seconds. After 21 cycles of dNTP flow, a PPi kernel is introduced, and then followed by another 21 cycles of dNTP flow. The end of the sequencing run is followed by a third PPi kernel. The total run time was 244 minutes. Reagent volumes required to complete this run are as follows: 500 mL of each wash solution, 100 mL of each nucleotide solution. During the run, all reagents were kept at room temperature. The temperature of the flow chamber and flow chamber inlet tubing is controlled at 30 °C and all reagents entering the flow chamber are pre-heated to 30 °C.

Example 5 Analysis of Soil Samples

Nucleic acid was extracted from organisms in the soil for analysis using the methods of the invention. Extraction was performed using a DNA extraction kit from Epicentre (Madison, WI, USA) following manufacturer's directions.

Briefly, 550 ul of Inhibitor Removal Resin was added to each empty Spin Column from Epicentre. The columns were centrifuged for one minute at 2000 x g to pack the column. The flow-through was removed and another 550 ul of Inhibitor Removal Resin was added to each column followed by centrifugation for 2 minutes at 2000 x g.

100 mg of soil was collected into a 1.5 ml tube and 250 ul of Soil DNA extraction buffer was added with 2 ul of Proteinase K. The solution was vortexed and 50 ul of Soil Lysis buffer was added and vortexed again. The tube was incubated at 65 C for 10 minutes and then centrifuged for 2 minutes at 1000 x g. 180 ul of the supernatant was transferred to a new tube and 60 ul of Protein Precipitation Reagent was added with thorough mixing by inverting the tube. The tube was incubated on ice for 8 minutes and centrifuged for 8 minutes at maximum speed. 100-150 ul of the supernatant was transferred directly onto the prepared Spin Column and the column was centrifuged for 2 minutes at 2000 x g into the 1.5 ml tube. The column was discarded and the eluate was collected. 6 ul of DNA Precipitation Solution was added to the eluate and the tube was mixed by a brief vortex. Following a 5 minute room temperature incubation, the tube was centrifuged for 5 minutes at maximum speed. Supernatant was removed and the pellet was washed with 500 ul of Pellet Wash Solution. The tube was inverted to mix the solution and then centrifuged for 3 minutes at maximum speed. Supernatant was removed and the wash step was repeated. Supernatant was removed again and the final pellet was resuspended in 300 ul of TE Buffer.

The DNA sample produced may be used for the methods of the invention including, at least, the methods for detecting nucleotide frequency at a locus.

## References

BioAnalyzer User Manual (Agilent): hypertext transfer protocol://world wide web.chem.agilent.com/temp/rad31B29/00033620.pdf

BioAnalyzer DNA and RNA LabChip Usage (Agilent): hypertext transfer protocol://world wide web.agilent.com/chem/labonachip

BioAnalyzer RNA 6000 Ladder (Ambion): hypertext transfer protocol://world wide web.ambion.com/techlib/spec/sp_7152.pdf

Biomagnetic Techniques in Molecular Biology, Technical Handbook, 3rd edition (Dynal, 1998): hypertext transfer protocol://world wide web.dynal.no/kunder/dynal/DynalPub36.nsf/cb927fbab127a0ad4125683b004b011c/4908f5b1a665858a41256adf005779f2/$FILE/Dynabeads M-280 Streptavidin.pdf.

Dinauer et al., 2000 Sequence-based typing of HLA class II DQB1. Tissue Antigens 55:364.

Garcia-Martinez, J., I. Bescos, et al. (2001). "RISSC: a novel database for ribosomal 16S-23S RNA genes spacer regions." Nucleic Acids Res 29(1): 178-80.

Grahn, N., M. Olofsson, et al. (2003). "Identification of mixed bacterial DNA contamination in broad-range PCR amplification of 16S rDNA V1 and V3 variable regions by pyrosequencing of cloned amplicons." FEMS Microbiol Lett 219(1): 87-91.

Hamilton, S.C., J.W. Farchaus and M.C. Davis. 2001. DNA polymerases as engines for biotechnology. BioTechniques 31:370.

Jonasson, J., M. Olofsson, et al. (2002). "Classification, identification and subtyping of bacteria based on pyrosequencing and signature matching of 16S rDNA fragments." Apmis 110(3): 263-72.

MinElute kit (QIAGEN): hypertext transfer protocol://world wide web.qiagen.com/literature/handbooks/minelute/1016839_HBMinElute_Prot_Gel.pdf.

Monstein, H., S. Nikpour-Badr, et al. (2001). "Rapid molecular identification and subtyping of Helicobacter pylori by pyrosequencing of the 16S rDNA variable V1 and V3 regions." FEMS Microbiol Lett 199(1): 103-7.

Norgaard et al., 1997 Sequencing-based typing of HLA-A locus using mRNA and a single locus-specific PCR followed by cycle-sequencing with AmpliTaq DNA polymerse. Tissue Antigens. 49:455-65.

Pollard, K. S. and M. J. van der Laan (2005). "Clsuter Analysis of Genomic Data with Applications in R." U.C. Berkeley Division of Biostatistics Working Paper Series # 167.

QiaQuick Spin Handbook (QIAGEN, 2001): hypertext transfer protocol://world wide web.qiagen.com/literature/handbooks/qqspin/1016893HBQQSpin_PCR_mc_prot.pdf.

Quick Ligation Kit (NEB): hypertext transfer protocol://world wide web.neb.com/neb/products/mod_enzymes/M2200.html.

Shimizu et al., 2002 Universal fluorescent labeling (UFL) method for automated microsatellite analysis. DNA Res. 9:173-78.

Steffens et al., 1997 Infrared fluorescent detection of PCR amplified gender identifying alleles. J. Forensic Sci. 42: 452-60.

Team, R. D. C. (2004). R: A language and environment for statistical computing. Vienna, Austria, R Foundation for Statistical Computing.

Tsang et al., 2004 Development of multiplex DNA electronic microarray using a universal adaptor system for detection of single nucleotide polymorphisms. Biotechniques 36:682-88.

## Claims

1.  A method for detecting sequence variants having a frequency of less than 5% in a nucleic acid population, the method comprising the steps of:

    (a) amplifying a polynucleotide segment common to said nucleic acid population with a pair of PCR nucleic acid primers that define a locus to produce a first population of amplicons each comprising said polynucleotide segment;
    (b) delivering the first population of amplicons into aqueous microreactors in a water-in-oil emulsion such that a plurality of the aqueous microreactors comprise (1) a single amplicon of the first population of amplicons (2) a single bead, and (3) amplification reaction solution containing reagents necessary to perform nucleic acid amplification;
    (c) clonally amplifying each member of said first population of amplicons by polymerase chain reaction to produce a plurality of populations of second amplicons wherein each population of second amplicons derives from one member of said first population of amplicons;
    (d) immobilizing said second amplicons to a plurality of the beads in the microreactors such that each bead comprises one population of said second amplicons;
    (e) breaking the emulsion to recover the beads from the microreactors;
    (f) determining in parallel a nucleic acid sequence for the second amplicons on each bead at a depth (i.e. number of individual sequence reads) of greater than 100 to produce a population of nucleic acid sequences; and
    (g) determining an incidence of each type of nucleotide at each position of said polynucleotide segment to detect said sequence variants in said nucleic acid population;

    wherein the method does not require prior knowledge of the nucleic acid sequence composition of the sequence variants,
    and wherein said nucleic acid primers are bipartite primers comprising a 5' region and a 3' region, wherein said 3' region is complementary to a region on said polynucleotide segment and wherein said 5' region is homologous to a sequencing primer or complement thereof.

2.  A method according to claim 1, wherein:

    step (e) comprises delivering the beads with bound second amplicons to an array of reaction chambers on a planar surface and performing a sequencing reaction simultaneously on the plurality of reaction chambers to determine a plurality of nucleic acid sequences corresponding to a plurality of alleles.

3.  A method according to claim 1 or 2 wherein the method comprises determining the nucleotide sequence of a polynucleotide region of interest at a depth (i.e. number of individual sequence reads) of greater than 1000.

4.  The method of claim 1 wherein said 5' region is homologous to a capture oligonucleotide or a complement thereof on said mobile solid support.

5.  The method of claim 1 wherein said bead has a diameter selected from the group consisting of between about 1 to about 500 microns, between about 5 to about 100 microns, between about 10 to about 30 microns and between about 15 to about 25 microns.

6.  The method of claim 1 wherein said bead comprise an oligonucleotide which hybridizes and immobilize said first population of amplicons, second amplicons, or both.

7.  The method of claim 1 wherein said step of determining a nucleic acid sequence is performed by delivering the plurality of mobile solid supports to an array of at least 10,000 reaction chambers on a planar surface, wherein a plurality of the reaction chambers comprise no more than a single mobile solid support; and determining a nucleic acid sequence of the amplicons on each said mobile solid support.

8.  The method of claim 1 wherein said step of determining a nucleic acid sequence is performed by pyrophosphate based sequencing.

9. The method of claim 1 wherein said nucleic acid population comprises DNA, RNA, cDNA or a combination thereof.

10. The method of claim 1 wherein the nucleic acid population is derived from a plurality of organisms.

11. The method of claim 1 wherein the nucleic acid population is derived from one organism.

12. The method of claim 11 wherein said nucleic acid population is derived from multiple tissue samples of said organism.

13. The method of claim 11 wherein said nucleic acid population is derived from a single tissue of said organism.

14. The method of claim 1 wherein the nucleic acid population is from a diseased tissue.

15. The method of claim 14 wherein said diseased tissue comprises tumor tissue.

16. The method of claim 1 wherein said nucleic acid population is derived from a bacterial culture, viral culture, or environmental sample.

17. The method of claim 1 wherein the first population of amplicons is 30 to 500 bases in length.

18. The method of claim 1 wherein said first population of amplicons comprises more than 1000 amplicons, more than 5000 amplicons, or more than 10000 amplicons.

19. The method of claim 1 wherein each of said solid supports binds at least 10,000 members of said plurality of second amplicons.

20. The method of claim 1 wherein the nucleic acid sequence of said polynucleotide segment is undetermined or partially undetermined before said method.

21. A method of identifying a distribution of organisms in a population comprising a plurality of different individual organisms, the method comprising using a nucleic acid sample from said population and comprising the steps of:

(a) determining sequence variants of a nucleic acid segment comprising a locus common to all organisms in said population using the method of claim 1, wherein each organism comprises a different nucleic acid sequence at said locus; and
(b) identifying the distribution of organisms in said population based on said population of nucleic acid sequences.

22. The method of claim 21 wherein said population is a population of organisms selected from the group consisting of bacteria, viruses, unicellular organisms, plants and yeasts.

23. A method for determining a composition of a tissue sample, the method comprising using a nucleic acid sample from said tissue sample and comprising the steps of:

(a) detecting sequence variants of a nucleic acid segment using the method of claim 1, wherein said segment comprises a locus common to all cells in said tissue sample and wherein each cell type comprises a different sequence variant at said locus; and
(b) determining the composition of said tissue sample from said nucleotide frequency.

**Patentansprüche**

1. Verfahren zum Detektieren von Sequenzvarianten mit einer Häufigkeit von weniger als 5% in einer Nukleinsäurepopulation, wobei das Verfahren die folgenden Schritte umfasst:

(a) Amplifizieren eines Polynukleotidsegments, das der Nukleinsäurepopulation gemeins ist, mit einem Paar von PCR-Nukleinsäureprimem, die einen Lokus um eine Population der jeweils das Polynukleotidsegment umfassenden Amplikons herzustellen definiert;

(b) Zuführen der ersten Population von Amplikons in wasserhaltigen Mikroreaktoren in einer Wasser-in-Öl-Emulsion, so dass eine Vielzahl der wasserhaltigen Mikroreaktoren (1) ein einzelnes Amplikon der ersten Population von Amplikons (2) ein einzelnes Kügelchen, und (3) Amplifikationsreaktionslösung enthaltend die Reagenzien, die notwendig sind, die Nukleinsäureamplifikation durchzuführen, umfasst;

(c) klonales Amplifizieren jedes Mitglieds der ersten Population der Amplikons durch Polymerasekettenreaktion um eine Vielzahl von Populationen zweiter Amplikons herzustellen, wobei jede Population zweiter Amplikons von einem Mitglied der ersten Population von Amplikons stammt;

(d) Immobilisieren der zweiten Amplikons auf einer Vielzahl der Kügelchen in der Mikroreaktoren, so dass jedes Kügelchen eine Population der zweiten Amplikons umfasst;

(e) Brechen der Emulsion um die Kügelchen aus den Mikroreaktoren zurückzugewinnen;

(f) Paralleles Bestimmen einer Nukleinsäuresequenz für die zweiten Amplikons auf jedem Kügelchen mit einer Tiefe (d.h, der Anzahl an individuellen Sequenzabschnitten) von größer als 100 um eine Population von Nukleinsäuresequenzen herzustellen; und

(g) Bestimmen einer Inzidenz jedes Typs an Nukleotid an jeder Position des Polynukleotidsegments um die Sequenzvarianten in der Nukleinsäurepopulation zu detektieren;

wobei das Verfahren kein vorheriges Wissen über die Nukleinsäuresequenzzusammensetzung der Sequenzvarianten benötigt;

und wobei die Nukleinsäureprimer zweigliedrige Primer, die eine 5'-Region und eine 3'-region umfassen sind, wobei die 3'-Region komplementär zu einer Region auf dem Polynukleotidsegment ist und wobei die 5'-Region homolog zu einem Sequenzierungsprimer oder komplementär dazu ist.

2. Verfahren gemäß Anspruch 1, wobei:

Schritt (e) das Zuführen der Kügelchen mit gebundenen zweiten Amplikons an eine Anordnung von Reaktionskammern auf einer planaren Oberfläche und das Durchführen einer Sequenzierungsreaktion gleichzeitig auf der Vielzahl der Reaktionskammern umfasst, um eine Vielzahl von Nukleinsäuresequenzen, die zu einer Vielzahl von Allelen korrespondiert, zu bestimmen.

3. Verfahren gemäß Anspruch 1 oder 2, wobei das Verfahren das Bestimmen der Nukleotidsequenz einer Polynukieotidregion von Interesse mit einer Tiefe (d.h. der Anzahl an individuellen Sequenzabschnitten) von größer als 1000 umfasst.

4. Verfahrens nach Anspruch 1, wobei die 5'-Region homolog zu einem Fänger-Oligonukleotid oder einem Komplement davon auf dem mobilen festen Trägern ist.

5. Verfahren nach Anspruch 1, wobei das Kügelchen einen Durchmeseer ausgewählt aus der Gruppe bestehend aus zwischen etwa 1 bis etwa 500 Mikrometer, zwischen etwa 5 bis etwa 100 Mikrometer, zwischen etwa 10 bis etwa 30 Mikrometer und zwischen etwa 15 bis etwa 25 Mikrometer ausweist.

6. Verfahren nach Anspruch 1, wobei das Kügelchen ein Oligonukleotid umfasst, das die erste Population von Amplikons, zweiten Amplikons, oder beide, hybridisiert und immobilisiert.

7. Verfahren nach Anspruch 1, wobei der Schritt des Bestimmens einer Nukleinsäurescquenz durch die Zuführung einer Vielzahl von mobilen festen Trägern an eine Anordnung von wenigstens 10.000 Reaktionskammern auf einer planaren Ebene durchgeführt wird, wobei eine Vielzahl der Reaktionskammern nicht mehr als einen einzelnen mobilen festen Träger umfasst; und Bestimmten einer Nukleinsäuresequenz des Amplikons auf jedem der mobilen festen Träger.

8. Verfahren nach Anspruch 1, wobei der Schritt des Bestimmens der Nukleinsäuresequenz durch pyrophosphatbasiertes Sequenzieren durchgeführt wird.

9. Verfahren nach Anspruch 1, wobei die Nukleinsäurepopulation DNA, RNA, cDNA oder eine Kombination davon umfasst.

10. Verfahren nach Anspruch 1, wobei die Nukleinsäurepopulation aus einer Vielzahl von Organismen stammt.

11. Verfahren nach Anspruch 1, wobei die Nukleinsäurepopulation aus einem Organismus stimmt.

**12.** Verfahren nach Anspruch 11, wobei die Nukleinsäurepopulation aus multiplen Gewebeproben des Organismus stimmt.

**13.** Verfahren nach Anspruch 11, wobei die Nukleinsäurepopulation aus einem einzigen Gewebe des Organismus stimmt.

**14.** Verfahren nach Anspruch 1, wobei die Nukleinsäurepopulation aus einem erkrankten Gewebe ist.

**15.** Verfahren nach Anspruch 14, wobei das erkrankte Gewebe Tumorgewebe umfasst.

**16.** Verfahren nach Anspruch 1, wobei die Nukleinsäurepopulation aus einer bakteriellen Kultur, einer viralen Kultur, oder einer Umweltprobe stimmt.

**17.** Verfahren nach Anspruch 1, wobei die population von Amplikons 30 bis 500 Basen lang ist.

**18.** Verfahren nach Anspruch 1, wobei die erste Population von Amplikons mehr als 1000 Amplikons, mehr als 5000 Amplikons, oder mehr als 10000 Amplikons umfasst.

**19.** Verfahren nach Anspruch 1, wobei jeder der festen Träger mindestens 10.000 Mitlieder der Vielzahl von zweiten Amplikons bindet.

**20.** Verfahren nach Anspruch 1, wobei die Nükleinsäuresequenz des Polynukleotidsegments vor dem Verfahren unbestimmt oder teilweise unbestimmt ist.

**21.** Verfahren zum Identifizieren einer Verteilung von Organismen in einer Population, die eine Vielzahl an verschiedenen individuellen Organsimen umfasst, wobei das Verfahren das Verwenden einer Nukleinsäureprobe aus der Population umfasst und die Schritte umfasst:

(a) Bestimmen von Sequevarianten eines Nukleinsäuresegments, das einen allen Organismen in der Population gemeinsamen Lokus umfasst, unter Verwendung des Verfahrens nach Anspruch 1, wobei jeder Organismus eine verschiedene Nukleinsäureseque an dem Lokus umfasst; und
(b) Identifizieren der Verteilung von Organsimen in der Population basiert auf der Population an Nukleinsäuresequenzen.

**22.** Verfahren nach Anspruch 21, wobei die Population eine Population von Organsimen ausgewählt aus der Gruppe bestehend aus Bakterien, Viren, einzelligen Organsimen, Pflanzen und Hefen ist.

**23.** Verfahren zum Bestimmen einer Zusammensetzung einer Gewebeprobe, wobei das Verfahren das Verwenden einer Nukleinsäureprobe aus der Gewebeprobe umfasst und die Schritte umfasst:

(a) Bestimmen von Sequenzvarianten eines Nukleinsäuresegments unter Verwendung des Verfahrens nach Anspruch 1, wobei das Segment einen allen Zellen in der Gewebeprobe gemeinsamen Lokus umfasst und wobei jeder Zelltyp eine verschiedene Sequenzvariante an dem Lokus umfasst; und
(b) Bestimmen der Zusammensetzung der Gewebeprobe aus der Nukleotidhäufigkeit,

**Revendications**

**1.** Procédé pour détecter des variants de séquence présentant une fréquence inférieure à 5 % dans une population d'acides nucléiques, lequel procédé comprend les étapes :

(a) d'amplification d'un segment polynucléotidique commun à ladite population d'acides nucléiques avec une paire d'amorces d'acide nucléique pour PCR qui définissent un locus pour produire une première population d'amplicons comprenant chacun ledit segment polynucléotidique ;
(b) d'introduction de la première population d'amplicons dans des microréacteurs en phase aqueuse, en émulsion eau-dans-l'huile, de sorte qu'une pluralité des microréacteurs en phase aqueuse comprenne (1) un seul amplicon de la première population d'amplicons, (2) une seule perle, et (3) une solution de réaction d'amplification contenant les réactifs nécessaires à la réalisation d'une amplification d'acide nucléique ;

(c) d'amplification par clonage de chaque membre de ladite première population d'amplicons par amplification en chaîne par polymérase pour produire une pluralité de populations de deuxièmes amplicons, chaque population de deuxièmes amplicons dérivant d'un membre donné de ladite première population d'amplicons ;

(d) d'immobilisation desdits deuxième amplicons sur une pluralité des perles dans les microréacteurs de sorte que chaque perle comprenne une seule population desdits deuxième 1amplicons ;

(e) de rupture de l'émulsion pour récupérer les perles à partir des microréacteurs ;

(f) de détermination en parallèle d'une séquence d'acide nucléique pour les deuxièmes amplicons sur chaque perle à une profondeur (c'est-à-dire le nombre de lectures de séquences individuelles) supérieure à 100 pour produire une population de séquences d'acide nucléique ; et

(g) de détermination de l'incidence de chaque type de nucléotide sur chaque position dudit segment polynucléotidique pour détecter lesdits variants de séquence dans ladite population d'acides nucléiques ;

lequel procédé ne requiert pas la connaissance préalable de la composition de séquence d'acide nucléique des variants de séquence,

dans lequel lesdites amorces d'acide nucléique sont des amorces bipartites comprenant une région 5' et une région 3', ladite région 3' étant complémentaire d'une région sur ledit segment polynucléotidique et ladite région 5' étant homologue d'une amorce de séquençage ou d'un complément de celle-ci.

2. Procédé selon la revendication 1, dans lequel l'étape (e) comprend la délivrance des perles avec des deuxièmes amplicons liés à un réseau de chambres réactionnelles sur une surface plane et la mise en oeuvre d'une réaction de séquençage simultanément sur la pluralité de chambres réactionnelles pour déterminer une pluralité de séquences d'acide nucléique correspondant à une pluralité d'allèles.

3. Procédé selon la revendication 1 ou 2, lequel procédé comprend la détermination de la séquence de nucléotides d'une région polynucléotidique présentant un intérêt à une profondeur (c'est-à-dire le nombre de lectures de séquences individuelles) supérieure à 1000.

4. Procédé selon la revendication 1, dans lequel ladite région 5' est homologue d'un oligonucléotide de capture ou d'un complément de celui-ci sur ledit support solide mobile.

5. Procédé selon la revendication 1, dans lequel ladite perle présente un diamètre choisi dans l'ensemble constitué par entre environ 1 et environ 500 micromètres, entre environ 5 et environ 100 micromètres, entre environ 10 et environ 30 micromètres, et entre environ 15 et environ 25 micromètres.

6. Procédé selon la revendication 1, dans lequel ladite perle comprend un oligonucléotide qui s'hybride avec et immobilise ladite première population d'amplicons, les deuxièmes amplicons, ou les deux.

7. Procédé selon la revendication 1, dans lequel ladite étape de détermination d'une séquence d'acide nucléique est effectuée par délivrance de la pluralité de supports solides mobiles à un réseau d'au moins 10 000 chambres réactionnelles sur une surface plane, une pluralité de chambres réactionnelles comprenant au plus un seul support solide mobile ; et détermination d'une séquence d'acide nucléique des amplicons sur chaque dit support solide mobile.

8. Procédé selon la revendication 1, dans lequel ladite étape de détermination d'une séquence d'acide nucléique est effectuée par séquençage basé sur le pyrophosphate.

9. Procédé selon la revendication 1, dans lequel ladite population d'acides nucléiques comprend des ADN, des ARN, des ADNc ou une combinaison de ceux-ci.

10. Procédé selon la revendication 1, dans lequel la population d'acides nucléiques dérive d'une pluralité d'organismes.

11. Procédé selon la revendication 1, dans lequel la population d'acides nucléiques dérive d'un seul organisme.

12. Procédé selon la revendication 11, dans lequel ladite population d'acides nucléiques dérive de multiples échantillons tissulaires dudit organisme.

13. Procédé selon la revendication 11, dans lequel ladite population d'acides nucléiques dérive d'un seul tissu dudit organisme.

**14.** Procédé selon la revendication 1, dans lequel la population d'acides nucléiques provient d'un tissu malade.

**15.** Procédé selon la revendication 14, dans lequel ledit tissu malade comprend un tissu tumoral.

**16.** Procédé selon la revendication 1, dans lequel ladite population d'acides nucléiques dérive d'une culture bactérienne, d'une culture virale, ou d'un échantillon environnemental.

**17.** Procédé selon la revendication 1, dans lequel la première population d'amplicons présente une longueur de 30 à 500 bases.

**18.** Procédé selon la revendication 1, dans lequel ladite première population d'amplicons comprend plus de 1000 amplicons, plus de 5000 amplicons, ou plus de 10000 amplicons.

**19.** Procédé selon la revendication 1, dans lequel chacun desdits supports solides se lie à au moins 10000 membres de ladite pluralité de deuxièmes amplicons.

**20.** Procédé selon la revendication 1, dans lequel la séquence d'acide nucléique dudit segment polynucléotidique est indéterminée ou partiellement indéterminée avant ledit procédé.

**21.** Procédé pour identifier une distribution d'organismes dans une population comprenant une pluralité de différents organismes individuels, lequel procédé comprend l'utilisation d'un échantillon d'acide nucléique provenant de ladite population et comprend les étapes :

(a) de détermination de variants de séquence d'un segment d'acide nucléique comprenant un locus commun à tous les organismes dans ladite population en utilisant le procédé de la revendication 1, chaque organisme comprenant une séquence d'acide nucléique différente au niveau dudit locus ; et
(b) d'identification de la distribution d'organismes dans ladite population sur la base de ladite population de séquences d'acide nucléique.

**22.** Procédé selon la revendication 21, dans lequel ladite population est une population d'organismes choisis dans l'ensemble constitué par les bactéries, les virus, les organismes unicellulaires, les plantes, et les levures.

**23.** Procédé pour déterminer la composition d'un échantillon tissulaire, lequel procédé comprend l'utilisation d'un échantillon d'acide nucléique provenant dudit échantillon tissulaire, et comprenant les étapes :

(a) de détection de variants de séquence d'un segment d'acide nucléique en utilisant le procédé de la revendication 1, ledit segment comprenant un locus commun à toutes les cellules dans ledit échantillon tissulaire et chaque type cellulaire comprenant un variant de séquence différent au niveau dudit locus ; et
(b) de détermination de la composition dudit échantillon tissulaire à partir de ladite fréquence de nucléotides.

Figure 1A

Figure 1B

Figure 2

Figure 3

Figure 4

Figure 5

A

B

Figure 6

Observed (Y) vs. Expected (X) Percentage C at position 62 of DD14 PCR Product

$y = 0.7009x - 0.0024$

◆ Percent C
—Linear (Percent C)

Figure 7

A

B

C

Figure 8

Figure 9

Population of first
polynucleotide
molecules (mix of
A and T variants)

Amplify polynucleotide
region of interest

Population of
second
polynucleotide
molecules (mix
of variants)

Sequencing of single
second polynucleotide
molecules

Sequences of
single second
polynucleotide
molecules
(variants
segregated)

AGCTGGCAACGTTGCC                AGCTGGCTACGTTGCC

Alignment

AGCTGGCAACGTTGCC
AGCTGGCTACGTTGCC

Polynucleotide
sequence
alignment
(variants
detected)

Figure 10

Population of first
polynucleotide
molecules (mix of A
and T variants)

Amplify polynucleotide
region of interest

Population of second
polynucleotide
molecules (mix of
variants)

Clonally amplify second
polynucleotide molecules

Populations of third
polynucleotide
molecules (variants
segregated)

Sequencing of
clonal amplification
products

AGCTGGCAACGTTGCC                    AGCTGGCTACGTTGCC

Sequences of
populations of third
polynucleotide
molecules (variants
segregated)

Alignment

AGCTGGCAACGTTGCC
AGCTGGCTACGTTGCC

Polynucleotide
sequence alignment
(variants detected)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004069849 A
- US 5908978 A
- US 5942392 A
- WO 9818967 A
- US 5814444 A
- US 767779 A
- US 0402570 W
- WO 04070007 A
- US 4683194 A
- US 4683195 A
- US 4683202 A
- US 20050100900 A
- US 20030022207 A
- US 20040096853 A
- WO 2005073410 A
- US 6432360 B
- US 6485944 B
- US 6511803 B
- US 6274320 B
- US 6258568 B
- US 6210891 B
- US 5989892 A
- WO 2005021786 A
- US 5622824 A
- US 6140053 A
- US 6828100 B
- US 6982146 B
- US 20030124594 A
- US 20060063171 A
- US 20060068401 A
- US 20050202444 A
- WO 9813523 A1
- US 20010024790 A
- US SN10768729 A
- US SN10767779 A
- US SN10767899 A
- US SN10767894 A
- US SN60476602 P
- US SN60476504 P
- US SN60443471 P
- US SN60476313 P
- US SN60476592 P
- US SN60465071 P
- US SN60497985 P
- US 10767899 A
- US 11104781 B

### Non-patent literature cited in the description

- **BENOMAR et al.** *Nat. Genet.,* 1995, vol. 10, 84-8
- **BLANTON et al.** *Genomics,* 1991, vol. 11, 857-69
- **CAVENEE et al.** *Nature,* 1983, vol. 305, 779-784
- **COLLINS et al.** *Proc. Natl. Acad Sci. USA,* 1996, vol. 93, 14771-14775
- **KOUFOS et al.** *Nature,* 1984, vol. 309, 170-172
- **LEGIUS et al.** *Nat. Genet.,* 1993, vol. 3, 122-126
- **DAVIS et al.** *Nature,* 1994, vol. 371, 130-136
- **TODD et al.** *Proc. Natl. Acad. Sci. USA,* 1995, vol. 92, 8560-8565
- **BOTSTEIN et al.** *Am. J. Hum. Genet.,* 1980, vol. 32, 314-31
- **NAKAMURA et al.** *Science,* 1987, vol. 235, 1616-22
- **DA MOTA et al.** *Eur. J. Immunogen,* 2002, vol. 29, 223-227
- **RICKERT et al.** *BioTechniques,* 2002, vol. 32, 592-603
- **AHMADIAN et al.** *Anal. BioChem.,* 2000, vol. 280, 103-110
- **THOMPSON ; THOMPSON.** Genetics in Medicine. W.B. Saunders Co, 1991
- Mapping the Human Genome. **STRACHAN.** Human Genome. Bios Scientific Publishers Ltd,
- **LATHROP.** *PNAS, USA,* 1984, vol. 81, 3443-3446
- **SMITH et al.** *Mathematical Tables for Research Workers in Human Genetics,* 1961
- **SMITH.** *Ann. Hum. Genet.,* 1968, vol. 32, 127-1500
- **SMITH et al.** *Breast Cancer Res. Terat.,* 1991, vol. 18 (1), 5-14
- **NOTOMI et al.** *Nucleic Acids Res.,* 2000, vol. 28 (12), E63
- DNA Amplification: Current Technologies and Applications. Horizon Bioscience, 2004
- **BARNES, W. M.** *Proc. Natl. Acad. Sci. USA,* 1994, vol. 91, 2216

- **SAIKI et al.** *Science,* 1985, vol. 230, 1350-1354
- **SAIKI et al.** *Nature,* 1986, vol. 324, 163-166
- **SCHARF et al.** *Science,* 1986, vol. 233, 1076-1078
- PCR Technology: Principles and Applications for DNA Amplification. Freeman Press, 1992
- PCR Protocols: A Guide to Methods and Applications. Academic Press, 1990
- **MATTILA et al.** *Nucleic Acids Res.,* 1991, vol. 19, 4967
- **ECKERT, K. A. ; KUNKEL, T. A.** PCR Methods and Applications. 1991, vol. 1, 17
- PCR. IRL Press
- **MITRA et al.** *Analyt. Biochem.,* 2003, vol. 320, 55-65
- **SANGER, F. et al.** *Proc. Natl. Acad. Sci. U.S.A.,* 1977, vol. 75, 5463-5467
- **MAXAM, A. M. ; GILBERT, W.** *Proc Natl Acad Sci USA,* 1977, vol. 74, 560-564
- **RONAGHI, M. et al.** *Science,* 1998, vol. 281, 363, 365
- **LYSOV, I. et al.** *Dokl Akad Nauk SSSR,* 1988, vol. 303, 1508-1511
- **BAINS W. ; SMITH G. C.** *J.TheorBiol,* 1988, vol. 135, 303-307
- **DRNANAC, R. et al.** *Genomics,* 1989, vol. 4, 114-128
- **KHRAPKO, K. R. et al.** *FEBS Lett,* 1989, vol. 256, 118-122
- **PEVZNER P. A.** *J Biomol Struct Dyn,* 1989, vol. 7, 63-73
- **SOUTHERN, E. M. et al.** *Genomics,* 1992, vol. 13, 1008-1017
- **METZGER ML.** *Genome Research,* 2005, 1767
- **RONAGHI et al.** *Anal. Biochem.,* 1996, vol. 242, 84-89
- **RONAGHI et al.** *Science,* 1998, vol. 281, 363-365
- **DINAUER et al.** Sequence-based typing of HLA class II DQB1. *Tissue Antigens,* 2000, vol. 55, 364
- **GARCIA-MARTINEZ, J. ; I. BESCOS et al.** RISSC: a novel database for ribosomal 16S-23S RNA genes spacer regions. *Nucleic Acids Res,* 2001, vol. 29 (1), 178-80
- **GRAHN, N. ; M. OLOFSSON et al.** Identification of mixed bacterial DNA contamination in broad-range PCR amplification of 16S rDNA V1 and V3 variable regions by pyrosequencing of cloned amplicons. *FEMS Microbiol Lett,* 2003, vol. 219 (1), 87-91
- **HAMILTON, S.C. ; J.W. FARCHAUS ; M.C. DAVIS.** DNA polymerases as engines for biotechnology. *BioTechniques,* 2001, vol. 31, 370
- **JONASSON, J. ; M. OLOFSSON et al.** Classification, identification and subtyping of bacteria based on pyrosequencing and signature matching of 16S rDNA fragments. *Apmis,* 2002, vol. 110 (3), 263-72
- **MONSTEIN, H. ; S. NIKPOUR-BADR et al.** Rapid molecular identification and subtyping of Helicobacter pylori by pyrosequencing of the 16S rDNA variable V1 and V3 regions. *FEMS Microbiol Lett,* 2001, vol. 199 (1), 103-7
- **NORGAARD et al.** Sequencing-based typing of HLA-A locus using mRNA and a single locus-specific PCR followed by cycle-sequencing with AmpliTaq DNA polymerse. *Tissue Antigens,* 1997, vol. 49, 455-65
- **POLLARD, K. S. ; M. J. VAN DER LAAN.** Clsuter Analysis of Genomic Data with Applications in R. *U.C. Berkeley Division of Biostatistics Working Paper Series # 167,* 2005
- **SHIMIZU et al.** Universal fluorescent labeling (UFL) method for automated microsatellite analysis. *DNA Res.,* 2002, vol. 9, 173-78
- **STEFFENS et al.** Infrared fluorescent detection of PCR amplified gender identifying alleles. *J. Forensic Sci.,* 1997, vol. 42, 452-60
- **TEAM, R. D. C.** R: A language and environment for statistical computing. R Foundation for Statistical Computing, 2004
- **TSANG et al.** Development of multiplex DNA electronic microarray using a universal adaptor system for detection of single nucleotide polymorphisms. *Biotechniques,* 2004, vol. 36, 682-88